# EUROPÄISCHE PATENTSCHRIFT

(11) **EP 1 366 189 B1**
(45) Veröffentlichungstag und Bekanntmachung des Hinweises auf die Patenterteilung: **15.08.2007**
(21) Anmeldenummer: 01978414.9
(22) Anmeldetag: 15.10.2001
(51) Int. Cl.: C12Q 1/68

(54) **NACHWEIS VON PATHOGENEN BAKTERIEN**
DETECTION OF PATHOGENIC BACTERIA
IDENTIFICATION DE BACTERIES PATHOGENES

(30) Priorität: 08.01.2001 DE 10100493
(43) Veröffentlichungstag der Anmeldung: 03.12.2003
(73) Patentinhaber: Biotecon Diagnostics GmbH, 10589 Berlin (DE)
(72) Erfinder: GRABOWSKI, Reiner, 37075 Göttingen (DE); GRÖNEWALD, Cordt, 13353 Berlin (DE); SCHNEIDER, Astrid, 13353 Berlin (DE); PARDIGOL, Andreas, 14480 Potsdam (DE); BERGHOF, Kornelia, 12355 Berlin (DE)
(74) Vertreter: Grünecker, Kinkeldey, Stockmair & Schwanhäusser Anwaltssozietät
(86) Internationale Anmeldenummer: PCT/EP2001/011901
(87) Internationale Veröffentlichungsnummer: WO 2002/053771

(56) Entgegenhaltungen:
- US-A- 5 652 102
- WELINDER-OLSSON C ET AL: "Improved microbiological techniques using the polymerase chain reaction and pulsed-field gel electrophoresis for diagnosis and follow-up of enterohaemorrhagic Escherichia coli infection." EUROPEAN JOURNAL OF CLINICAL MICROBIOLOGY & INFECTIOUS DISEASES, Bd. 19, Nr. 11, November 2000 (2000-11), Seiten 843-851, XP002228587 ISSN: 0934-9723
- KONG R Y C ET AL: "A sensitive and versatile multiplex PCR system for the rapid detection of enterotoxigenic (ETEC), enterohaemorrhagic (EHEC) and enteropathogenic (EPEC) strains of Escherichia coli." MARINE POLLUTION BULLETIN, Bd. 38, Nr. 12, Dezember 1999 (1999-12), Seiten 1207-1215, XP002228588 ISSN: 0025-326X
- GANNON V P J ET AL: "USE OF THE FLAGELLAR H7 GENE AS A TARGET IN MULTIPLEX PCR ASSAYS AND IMPROVED SPECIFICITY IN IDENTIFICATION OF ENTEROHEMORRHAGIC ESCHERICHIA COLI STRAINS" JOURNAL OF CLINICAL MICROBIOLOGY, WASHINGTON, DC, US, Bd. 35, Nr. 3, Mai 1997 (1997-05), Seiten 656-662, XP000957649 ISSN: 0095-1137
- DENG M Y ET AL: "A MULTIPLEX PCR FOR RAPID IDENTIFICATION OF SHIGA-LIKE TOXIN-PRODUCING ESCHERICHIA COLI O157:H7 ISOLATED FROM FOODS" JOURNAL OF FOOD PROTECTION, DES MOINES, IO, US, Bd. 59, Nr. 6, 1996, Seiten 570-576, XP001004598 ISSN: 0362-028X
- FAGAN P K ET AL: "DETECTION OF SHIGA-LIKE TOXIN (STX1 AND STX2), INTIMIN (EAEA), AND ENTEROHEMORRHAGIC ESCHERICHIA COLI (EHEC) HEMOLYSIN (EHEC HLYA) GENES IN ANIMAL FECES BY MULTIPLEX PCR" APPLIED AND ENVIRONMENTAL MICROBIOLOGY, WASHINGTON,DC, US, Bd. 65, Nr. 2, Februar 1999 (1999-02), Seiten 868-872, XP001001377 ISSN: 0099-2240
- PATON A W ET AL: "DETECTION AND CHARACTERIZATION OF SHIGA TOXIGENIC ESCHERICHIA COLI BY USING MULTIPLEX PCR ASSAYS FOR STX1, STX2, EAEA, ENTEROHEMORRHAGIC E. COLI HLYA, RFBA, RFB0111, AND RFB0157" JOURNAL OF CLINICAL MICROBIOLOGY, WASHINGTON, DC, US, Bd. 36, Nr. 2, 1998, Seiten 598-602, XP000908789 ISSN: 0095-1137
- STEPHAN R ET AL: "Prevalence and characteristics of verotoxin-producing Escherichia coli (VTEC) in stool samples from asymptomatic human carriers working in the meat processing industry in Switzerland." JOURNAL OF APPLIED MICROBIOLOGY., Bd. 88, Nr. 2, Februar 2000 (2000-02), Seiten 335-341, XP002228589 ISSN: 1364-5072
- DATABASE EMBL [Online] EBI3. März 1993 (1993-03-03) PATON ET AL.: "Escherichia coli Shiga-like toxin type-I alpha-subunit and beta-subunit genes, complete cds." retrieved from HTTP://WWW.EBI.AC.UK Database accession no. L04539 XP002228590

### SEQUENZPROTOKOLL

<110> Biotecon Diagnostics
<120> Nachweis von pathogenen Bakterien
<130> 1
<140> 1
   <141> 2000-04-30
<160> 98
<170> PatentIn Ver. 2.1
<210> 1
   <211> 18
   <212> DNA
   <213> Escherichia coli
<400> 1
   ctggggaagg ttgagtag 18
<210> 2
   <211> 20
   <212> DNA
   <213> Escherichia coli
<400> 2
   gtcctgcctg aytatcatgg 20
<210> 3
   <211> 21
   <212> DNA
   <213> Escherichia coli
<400> 3
   acaagactct gttcgtgtag g 21
<210> 4
   <211> 27
   <212> DNA
   <213> Escherichia coli
<400> 4
   aagaatttct tttgraagyr ttaatgc 27
<210> 5
   <211> 28
   <212> DNA
   <213> Escherichia coli
<400> 5
   aattctgggw agcgtggcat taatactg 28
<210> 6
   <211> 20
   <212> DNA
   <213> Escherichia coli
<400> 6
   cccactttaa ctgtaaaggt 20
<210> 7
   <211> 29
   <212> DNA
   <213> Escherichia coli
<400> 7
   cgtcatcatt atattttgta tactccacc 29
<210> 8
   <211> 22
   <212> DNA
   <213> Escherichia coli
<400> 8
   cacttgctga aaaaaatgaa ag 22
<210> 9
   <211> 26
   <212> DNA
   <213> Escherichia coli
<400> 9
   agcgtggcat taatactgaa ttgtca 26
<210> 10
   <211> 25
   <212> DNA
   <213> Escherichia coli
<400> 10
   atcatgcatc gcgagttgcc agaat 5
<210> 11
   <211> 25
   <212> DNA
   <213> Escherichia coli
<400> 11
   atcatgcatc gcgagttgcc agaat 25
<210> 12
   <211> 35
   <212> DNA
   <213> Escherichia coli
<400> 12
   ttcgtgtwgg aagaatttct tttgraagyr ttaat 35
<210> 13
   <211> 33
   <212> DNA
   <213> Escherichia coli
<400> 13
   atgagtttcc ttctatgtgy ccggyagatg gaa 33
<210> 14
   <211> 37
   <212> DNA
   <213> Escherichia coli
<400> 14
   tccgtgggat tacgcacaat aaaatatttg tgggatt 37
<210> 15
   <211> 32
   <212> DNA
   <213> Escherichia coli
<400> 15
   aaayattatt aatagctgca tcrctttcat tt 32
<210> 16
   <211> 34
   <212> DNA
   <213> Escherichia coli
<400> 16
   ttcagcaagt gygctggckr cgccwgattc tgta 34
<210> 17
   <211> 33
   <212> DNA
   <213> Escherichia coli
<400> 17
   actggraagg tggagtatac aaaatataat gat 33
<210> 18
   <211> 19
   <212> DNA
   <213> Escherichia coli
<400> 18
   ggcactgtct gaaactgct 19
<210> 19
   <211> 20
   <212> DNA
   <213> Escherichia coli
<400> 19
   gaaactgctc ctgtktatac 20
<210> 20
   <211> 19
   <212> DNA
   <213> Escherichia coli
<400> 20
   gatgacrccg gragamgtg 19
<210> 21
   <211> 27
   <212> DNA
   <213> Escherichia coli
<400> 21
   ctgaactggg ggmgaatcag caatgtg 27
<210> 22
   <211> 18
   <212> DNA
   <213> Escherichia coli
<400> 22
   ygccattgca ttaacaga 18
<210> 23
   <211> 23
   <212> DNA
   <213> Escherichia coli
<400> 23
   gcwgckgtat tactttccca taa 23
<210> 24
   <211> 32
   <212> DNA
   <213> Escherichia coli
<400> 24
   ggcctgtcgc cagttatctg acattctggt tg 32
<210> 25
   <211> 32
   <212> DNA
   <213> Escherichia coli
<400> 25
   ggcctgtcgc cagttatctg acattctggt tg 32
<210> 26
   <211> 19
   <212> DNA
   <213> Escherichia coli
<400> 26
   ggcgctgtct gaggcatct 19
<210> 27
   <211> 20
   <212> DNA
   <213> Escherichia coli
<400> 27
   gaggcatctc cgctttatac 20
<210> 28
   <211> 19
   <212> DNA
   <213> Escherichia coli
<400> 28
   aatgacggct caggatgtt 19
<210> 29
   <211> 27
   <212> DNA
   <213> Escherichia coli
<400> 29
   ctgaactggg gaagaataag taatgtt 27
<210> 30
   <211> 30
   <212> DNA
   <213> Escherichia coli
<400> 30
   gcagcgattg tattcgcttc ccacaaaaca 30
<210> 31
   <211> 32
   <212> DNA
   <213> Escherichia coli
<400> 31
   gccctgtctc caacaatctg gcattctgtt tt 32
<210> 32
   <211> 21
   <212> DNA
   <213> Escherichia coli
<400> 32
   ctgtttttgg ctcacggaac g 21
<210> 33
   <211> 22
   <212> DNA
   <213> Escherichia coli
<400> 33
   cgccatggaa ttagcagaaa ag 22
<210> 34
   <211> 21
   <212> DNA
   <213> Escherichia coli
<400> 34
   ccccagttca gwgtgaggtc c 21
<210> 35
   <211> 21
   <212> DNA
   <213> Escherichia coli
<400> 35
   ccggaagcac attgctgatt c 21
<210> 36
   <211> 34
   <212> DNA
   <213> Escherichia coli
<400> 36
   gaatatcctt taataatata tcagcgatac tkgg 34
<210> 37
   <211> 33
   <212> DNA
   <213> Escherichia coli
<400> 37
   wgtggcsgtt atactgaatt gycatcatca ggg 33
<210> 38
   <211> 28
   <212> DNA
   <213> Escherichia coli
<400> 38
   cgttcygttc gckccgtgaa tgaagaka 28
<210> 39
   <211> 32
   <212> DNA
   <213> Escherichia coli
<400> 39
   caaccagaat gtcagataac tggcgacagg cc 32
<210> 40
   <211> 21
   <212> DNA
   <213> Escherichia coli
<400> 40
   ccccagttca gggtaaggtc a 21
<210> 41
   <211> 21
   <212> DNA
   <213> Escherichia coli
<400> 41
   ctggaagaac attacttatt c 21
<210> 42
   <211> 35
   <212> DNA
   <213> Escherichia coli
<400> 42
   aggatatctt ttaatagtct ttctgcgatt ctcgg 35
<210> 43
   <211> 33
   <212> DNA
   <213> Escherichia coli
<400> 43
   tgttgcggtc atccttaatt gccactcaac cgg 33
<210> 44
   <211> 29
   <212> DNA
   <213> Escherichia coli
<400> 44
   ttattcagtt cgttccgtga gccaaaaac 29
<210> 45
   <211> 32
   <212> DNA
   <213> Escherichia coli
<400> 45
   aaaacagaat gccagattgt tggagacagg gc 32
<210> 46
   <211> 20
   <212> DNA
   <213> Escherichia coli
<220>
   <221> variation
   <222> (9)
   <223> n = Inosin
<400> 46
   catgctgcnt ttttagaaga 20
<210> 47
   <211> 20
   <212> DNA
   <213> Escherichia coli
<400> 47
   catgctgcrt ttttagaaga 20
<210> 48
   <211> 24
   <212> DNA
   <213> Escherichia coli
<220>
   <221> variation
   <222> (9)
   <223> n = Inosin
<400> 48
   catgctgcnt ttttagaaga ctct 24
<210> 49
   <211> 24
   <212> DNA
   <213> Escherichia coli
<400> 49
   catgctgcrt ttttagaaga ctct 24
<210> 50
   <211> 24
   <212> DNA
   <213> Escherichia coli
<400> 50
   aatgaatggg aaaaggagca tggc 24
<210> 51
   <211> 23
   <212> DNA
   <213> Escherichia coli
<400> 51
   ctctctgtct ttgcttgctg att 23
<210> 52
   <211> 30
   <212> DNA
   <213> Escherichia coli
<400> 52
   ctcgtcagca tgcagtagaa agagcagtcg 30
<210> 53
   <211> 32
   <212> DNA
   <213> Escherichia coli
<400> 53
   cattgggatg agaagatcgg tgaacttgca gg 32
<210> 54
   <211> 21
   <212> DNA
   <213> Escherichia coli
<400> 54
   cgtctttatc tccgagytca g 21
<210> 55
   <211> 25
   <212> DNA
   <213> Escherichia coli
<400> 55
   acatcgtctt tatctccgag ytcag 25
<210> 56
   <211> 32
   <212> DNA
   <213> Escherichia coli
<400> 56
   tttaccaaca tccgtcttat tataagatac gg 32
<210> 57
   <211> 22
   <212> DNA
   <213> Escherichia coli
<400> 57
   ccttcaccag caaatacttc tg 22
<210> 58
   <211> 22
   <212> DNA
   <213> Escherichia coli
<400> 58
   tgagcctgct ccagaataaa cc 22
<210> 59
   <211> 23
   <212> DNA
   <213> Escherichia coli
<400> 59
   tcaattttga ataatcatat aca 23
<210> 60
   <211> 40
   <212> DNA
   <213> Escherichia coli
<400> 60
   agagaaagaa aacagagtgg taaatatgaa tatatgacat 40
<210> 61
   <211> 38
   <212> DNA
   <213> Escherichia coli
<400> 61
   tcttattgta aatggtaagg atacatggtc tgtaaaag 38
<210> 62
   <211> 41
   <212> DNA
   <213> Escherichia coli
<400> 62
   gggaccatag acctttcaac aggtaatgta tcaagtgttt t 41
<210> 63
   <211> 37
   <212> DNA
   <213> Escherichia coli
<400> 63
   acatttataa caccaacatt taccccagga gaagaag 37
<210> 64
   <211> 42
   <212> DNA
   <213> Escherichia coli
<400> 64
   ggcatatatt aattatctgg aaaatggagg gcttttagag gc 42
<210> 65
   <211> 37
   <212> DNA
   <213> Escherichia coli
<400> 65
   caaccgaagg agtttacaca acaagtgttt gatcctc 37
<210> 66
   <211> 35
   <212> DNA
   <213> Escherichia coli
<400> 66
   cattgggatg agaagatcgg tgaacttgca ggcat 35
<210> 67
   <211 > 36
   <212> DNA
   <213> Escherichia coli
<400> 67
   aacccgtaat gctgatcgca gtcagagtgg taaggc 36
<210> 68
   <211> 21
   <212> DNA
   <213> Escherichia coli
<400> 68
   ggcctggtta caacattatg g 21
<210> 69
   <211> 25
   <212> DNA
   <213> Escherichia coli
<400> 69
   acgcgaaaga taccgctctt ggtat 25
<210> 70
   <211> 21
   <212> DNA
   <213> Escherichia coli
<400> 70
   ccaggcttcg tcacagttgc a 21
<210> 71
   <211> 24
   <212> DNA
   <213> Escherichia coli
<400> 71
   ggaacggcag aggttaatct gcag 24
<210> 72
   <211> 26
   <212> DNA
   <213> Escherichia coli
<400> 72
   agtggtaata actttgacgg tagttc 26
<210> 73
   <211> 18
   <212> DNA
   <213> Escherichia coli
<400> 73
   atccccatcg tcaccaga 18
<210> 74
   <211> 21
   <212> DNA
   <213> Escherichia coli
<400> 74
   aacattatca ccataatact g 21
<210> 75
   <211> 23
   <212> DNA
   <213> Escherichia coli
<400> 75
   tagtttacac caacggtcgc cgc 23
<210> 76
   <211> 21
   <212> DNA
   <213> Escherichia coli
<400> 76
   cattacccgt accatgacgg t 21
<210> 77
   <211> 27
   <212> DNA
   <213> Escherichia coli
<400> 77
   cggaactgca ttgagtaaag gagatca 27
<210> 78
   <211> 31
   <212> DNA
   <213> Escherichia coli
<400> 78
   tccagtgaac taccgtcaaa gttatyacca c 31
<210> 79
   <211> 31
   <212> DNA
   <213> Escherichia coli
<400> 79
   tccagtgaac taccgtcaaa gttatyacca c 31
<210> 80
   <211> 28
   <212> DNA
   <213> Escherichia coli
<400> 80
   atgttgggct ataacgtctt cattgatc 28
<210> 81
   <211> 26
   <212> DNA
   <213> Escherichia coli
<400> 81
   aggatttttc tggtgataat acccgt 26
<210> 82
   <211> 42
   <212> DNA
   <213> Escherichia coli
<400> 82
   aggtattggt ggcgaatact ggcgagacta tttcaaaagt ag 42
<210> 83
   <211> 41
   <212> DNA
   <213> Escherichia coli
<400> 83
   ttaacggcta tttccgcatg agcggctggc atgagtcata c 41
<210> 84
   <211> 22
   <212> DNA
   <213> Escherichia coli
<400> 84
   cgggtcaggt aattgcacag ta 22
<210> 85
   <211> 22
   <212> DNA
   <213> Escherichia coli
<400> 85
   cgggtcaggt gattgcacag ta 22
<210> 86
   <211> 22
   <212> DNA
   <213> Escherichia coli
<400> 86
   cgggtcaggt gattgcacaa ta 22
<210> 87
   <211> 22
   <212> DNA
   <213> Escherichia coli
<400> 87
   cgggtcaggt aattgcacaa ta 22
<210> 88
   <211> 22
   <212> DNA
   <213> Escherichia coli
<400> 88
   gcaacagttc agcaaagtcc at 22
<210> 89
   <211> 21
   <212> DNA
   <213> Escherichia coli
<400> 89
   cggtgaagcc accgacatcg t 21
<210> 90
   <211> 24
   <212> DNA
   <213> Escherichia coli
<400> 90 24
   tggcaggttc cggccttcac tctc 24
<210> 91
   <211> 17
   <212> DNA
   <213> Escherichia coli
<400> 91 17
   aagccaccga catcgtg 17
<210> 92
   <211> 17
   <212> DNA
   <213> Escherichia coli
<400> 92 17
   aagccactga catcgtg 17
<210> 93
   <211> 31
   <212> DNA
   <213> Escherichia coli
<400> 93
   tccagtgaac taccgtcaaa gttatyacca c 31
<210> 94
   <211> 37
   <212> DNA
   <213> Escherichia coli
<400> 94
   ccagcatktt ttcggaatca tagaacggta ataagaa 37
<210> 95
   <211> 27
   <212> DNA
   <213> Escherichia coli
<400> 95
   attaayrctt ycaaaagaaa ttcttcc 27
<210> 96
   <211> 28
   <212> DNA
   <213> Escherichia coli
<400> 96
   cagtattaat gccacgctwc ccagaatt 28
<210> 97
   <211> 25
   <212> DNA
   <213> Escherichia coli
<400> 97
   ccttctatgt gyccggyaga tggaa 25
<210> 98
   <211> 20
   <212> DNA
   <213> Escherichia coli
<400> 98
   tscgtgggat tacgcacaat 20

## Beschreibung

Die vorliegende Erfindung betrifft ein Verfahren zum Nachweis von EHEC-Bakterien sowie hierfür geeignete Oligonukleotide.

Im Zeitalter weltweiten Transports und rationeller Verarbeitungsmethoden wächst die Bedeutung der durch Lebensmittel übertragenen pathogenen Bakterien. Häufig werden Rohstoffe aus vielen Landesteilen an einem zentralen Ort gesammelt, durchmischt und zu einem bestimmten Lebensmittel verarbeitet. Wenn eines der Rohprodukte Träger eines pathogenen Keimes war, dann kann sich dieser während des Produktionsprozesses vermehren und zur Kontamination einer großen Lebensmittelcharge führen.

*Escherichia coli* ist in diesem Zusammenhang als sehr wichtiger pathogener Keim aufgetreten. Hinter Campylobacter und Salmonella ist es der dritthäufigste lebensmittelverderbende Keim. Das Bakterium kommt normaler Weise als harmloser Kommensale im Darm des Menschen vor. Es kann jedoch jedoch bestimmte Pathogenitätsgene aufnehmen und dann zu einer todbringenden Gefahr werden. So sind eine ganze Reihe von *E*. *coli*-Subtypen charakterisiert worden, die ein hohes pathogenes Potential besitzen. Dazu zählen die Shigella-Stämme, die eigentlich systematisch *E*. *coli* zuzuordnen sind. Des weiteren zu nennen seien EPEC (enteropathogene *E. coli*), die insbesondere bei Säuglingen Durchfallerkrankungen verursachen, ETEC (enterotoxinogene *E. coli*), die extrazelluläre hitzestabile und hitzelabile Toxine bilden und im wesentlichen für die Reisediarrhoe verantwortlich sind und EIEC, die in Darmschleimhautzellen eindringen und eine Bakterienruhr verursachen.

Eine besonders gefährliche Gruppe pathogener *E. coli*-Stämme sind die EHECs (enterohämorrhagische *E*. *coli*). Zur Gruppe der EHECs ist auch der besonders häufig auftretende Serotyp O157:H7 zu zählen. Dieser, wie auch die übrigen Mitglieder der Gruppe, können das hämolytisch-urämische Syndrom (HUS) verursachen, das zum Tode führen kann. HUS ist begleitet von blutigem Durchfall und akutem Nierenversagen.

Das endemische Vorkommen der EHECs beschränkt sich in der Natur weitgehend auf Rinder, wenn auch andere Quellen, insbesondere Schweine als Reservoir dokumentiert sind. Infolge dessen sind verarbeitete Rindfleischprodukte, unter diesen besonders das Hackfleisch, häufig mit EHECs kontaminiert. In einigen Lebensmitteluntersuchungen waren mehr als 50% der Hackfleischproben EHEC-positiv. Es wurden in den letzten Jahren auch andere Lebensmittel, wie Salate, Radieschen, Milch und Milchprodukte als EHEC-Quellen identifiziert.

In den USA kamen in der letzten Dekade jährlich mehr als 20000 *E*. *coli* O157:H7-Infektionen vor (Boyce et al. 1995, N.Engl. J. Med. 333, 364-368), von denen ca. 250 tödlich ausgingen. Aufgrund mangelnder Diagnostik können die realen Zahlen jedoch noch viel höher liegen. In Europa und Japan werden *E*. *coli* O157:H7-Infektionen primär im Sommer berichtet. Demgegenüber sind in der südlichen Hemisphäre nicht-O157 EHEC-Serotypen von besonders großer Bedeutung.

Das pathogene Potential eines EHEC-Stammes wird durch seine Pathogenitätsfaktoren bestimmt. So ist das Vorkommen von Slt-Genen (Shiga-like toxin oder vtx = Verotoxingen) eine notwendige, jedoch keine hinreichende Voraussetzung für Pathogenität. Zusätzlich sind andere Faktoren charakterisiert worden (Nataro und Kaper 1998, Clin.Microb.Rev. 11, 142-201), die notwendig sind, um den Wirt zu infizieren. Viele dieser Faktoren sind nicht konstant im Genom kodiert, sondern liegen auf transferierbaren Plasmiden oder in Phagengenomen. Deshalb kann auch die Ausstattung von EHEC-Stämmen mit Pathogenitätsfaktoren einer zeitlichen Variabilität unterliegen.

Der sichere diagnostische Nachweis von EHEC-Stämmen mit den bekannten Methoden verursacht große Probleme. So sind mikrobiologische Verfahren kaum geeignet einen sicheren Nachweis zu führen. Stoffwechselphysiologische Unterschiede zwischen apathogenen *E*. *coli* und pathogenen EHEC-Stämmen sind kaum vorhanden. Der für *E*. *coli* O157:H7 häufig charakteristische Defekt des uidA-Gens (β-Glucuronidase) (Cebula et al. 1995, J. Clin. Microb.33, 248-250), ist kein sicheres Merkmal der EHEC-Gruppe. Aus diesem Grunde müssen diagnostische Methoden auf molekularbiologische Merkmale zurückgreifen.

Eine in der Vergangenheit häufig verwendete Methode war die Serotypisierung durch einen ELlSA. Diese weist jedoch viele Nachteile auf, da sie relativ zeitaufwendig ist und viele Arbeitsschritte erfordert. Außerdem ist ihre Sensitivität für viele diagnostische Anwendungen nicht ausreichend. Darüber hinaus ist der Serotyp alleine kein hinreichendes Merkmal für Pathogenität.

Eine weitere Methode *E. coli*-Stämme zu differenzieren besteht darin DNA-Sequenzunterschiede zu untersuchen. Die Vorgehensweise basiert insbesondere darauf, dass pathogene Stämme bestimmte Toxingene besitzen. So können z.B. die Shiga-ähnlichen Toxingene (Shiga-like toxins, slt, oder Verotoxingene, vtx) direkt nachgewiesen werden (Takeshi et al. 1997, Microb. Immun. 41, 819-822, Paton and Paton 1999, J. Clin. Microb. 37, 3362-3365). Die PCR kann angewendet werden, um Teile des Gens zu amplifizieren. Diese Fragmente können sichtbar gemacht werden, so dass sie als diagnostisches Merkmal dienen.

Der Nachteil dieses Verfahrens besteht darin, dass Slt-Gene keine ausreichende Voraussetzung für Pathogenität sind. Weitere DNA-Sequenz-Merkmale sind notwendig, um eine eindeutige Korrelation zwischen Genotyp und Pathogenität herzustellen. Die *E*. *coli*-Stämme, die Slt-Gene besitzen werden als VTEC (Verotoxin bildende *E*. *coli* oder STEC) bezeichnet Sie bilden somit eine größere Gruppe als die EHEC.

Es wurden auch andere genetische Marker für EHEC oder Untergruppen aus diesen erprobt Hierzu zählen das fimA-Gen (Li et al. 1997, Mol. Cell. Probes, 11, 397-406) und das fliC-Gen (Fields et al. 1997, J. Clin. Microb. 35, 1066-1070). Sie haben jedoch alle den Nachteil nur einen Teil der EHEC-Gruppe abzubilden.

Da die EHEC-Gruppe phylogenetisch keine systematische Einheit bildet, stellt sich die schwierige Aufgabe, genetische Polymorphismen zu finden, durch welche sie eindeutig charakterisiert ist Diese Polymorphismen sollten zudem so zuverlässig sein, dass sie auch Heterogenitäten und genetische lnstabilitäten innerhalb der EHEC-Gruppe erfassen. Neben dem spezifischen Nachweis sollten sie einen möglichst sensitiven Nachweis der EHEC erlauben.

US 5,652,102, PATON ET AL, JOURNAL OF CLINICAL MICROBIOLOGY, WASHINGTON, DC, US, Bd. 36, Nr. 2, 1998, Seiten 598-602 und FAGAN ET AL: ", APPLIED AND ENVIRONMENTAL MICROBIOLOGY, WASHINGTON,DC, US, Bd. 65, Nr. 2, Februar 1999 (1999-02), Seiten 868-872 offenbaren verschiedene Verfahren zum EHEC-Nachweis mittels Sequenzen aus den Slt2-, eae- und hlyA-Genen.

Es gibt bereits einige Nachweissysteme für als EHECs klassifizierte *E*. *coli.* Soweit diese auf einer immunologischen Detektion beruhen, ist ihre Sensitivität jedoch nicht ausreichend. Außerdem sind Antikörpernachweise sehr empfindlich gegenüber externen Verunreinigungen. Extrakte aus Lebensmittel sind sehr problematisch, da sie die antigenen Oberflächen der Bakterien verdecken oder gar zerstören. Soweit dennoch einige Oberflächenantigene zur Exposition gelangen sind es häufig zu wenige, um mit ausreichender Sensitivität einen sicheren Nachweis zu gewährleisten.

Der vorliegenden Erfindung lag das technische Problem zugrunde, ein Verfahren anzugeben, das den sicheren Nachweis von EHEC-Bakterien in einer beliebigen Probe gewährleistet und das möglichst geringen Beeinträchtigungen durch weitere Probenbestandteile, wie PCR-Inhibitoren, der DNA nicht-pathogener Bakterien oder dem Quenching-Phänomen (s. Kapitel Optimierung der Online-PCR") unterliegt Ferner lag der vorliegenden Erfindung das weitere Problem zugrunde, die für den EHEC-Nachweis erforderlichen Mittel zur Verfügung zu stellen.

Das erste Problem wird erfindungsgemäß gelöst durch
**Ausführungsform I:**
Verfahren zum Nachweis von EHEC-Bakterien in einer Probe, umfassend den Schritt:
Nachweisen des Vorkommens einer Nukleinsäuresequenz aus dem Slt-Locus in Kombination mit einer Sequenz aus dem eae-Locus und/oder dem hylA-Locus in der Probe,
wobei zumindest zwei Vorwärtsprimer/Rückwärtsprimer-Paare, welche SEQ ID Nrn: 1 und 6 (Kategorie A) oder jeweils entsprechende Derivate davon, bzw. SEQ ID Nrn: 18 und 22 (Kategorie B) oder jeweils entsprechende Derivate davon sind, kombiniert werden mit zumindest einem Vorwärtsprimer und zumindest einem Rückwärtsprimer aus einer der Kategorien D, d.h. ausgewählt aus SEQ ID Nr 46 oder entsprechenden Derivaten davon (Vorwärtsprimer) bzw. SEQ ID Nr: 54 oder entsprechenden Derivaten davon (Rückwärtsprimer), und E, d.h. ausgewählt aus SEQ ID Nm: 68, oder entsprechenden Derivaten davon (Vorwärtsprimer) bzw. SEQ ID Nrn: 73 oder entsprechenden Derivate davon (Rückwärtsprimer),
wobei die entsprechenden Derivate
a) zumindest 80% identisch zur jeweiligen oben aufgeführten Sequenz sind, oder
b) sich durch genau einen Basenaustausch, genau eine Insertion, genau eine Deletion oder genau eine Addition von einer jeweiligen Sequenz unterscheiden; und
wobei der gleichzeitige Nachweis von Sequenzen
i) aus dem Slt-Locus und dem eae-Locus oder
ii) aus dem Slt-Locus und dem hlyA-Locus
in der Probe eine ausreichend hohe Aussagekraft für den EHEC Nachweis besitzt.

Das zweite Problem wird erfindungsgemäß gelöst durch
**Ausführungsform II:**
Eine Kombination von Oligonukleotiden umfassend
zumindest zwei Primerpaare von
Vorwärts- und Rückwärtsprimern, ausgewählt aus
A) SEQ ID Nrn:1 und 6 (Kategorie A) oder jeweils entsprechenden Derivaten davon, und
B) SEQ ID NOS: 18 und 22 (Kategorie B) oder jeweils entsprechenden Derivaten davon, und
zumindest ein Primerpaar von Vorwärts- und Rückwärtsprimern, ausgewählt aus einer der Kategorien
i) D, d.h. ausgewählt aus SEQ ID Nrn: 46 oder entsprechenden Derivaten davon (Vorwärtsprimer), bzw. SEQ ID Nrn: 54 oder entsprechenden Derivaten davon (Rückwärtsprimer), und
ii) E, d.h. ausgewählt aus SEQ ID Nr: 68 oder entsprechenden Derivaten davon (Vorwärtsprimer), bzw. SEQ ID Nr: 73 oder entsprechenden Derivaten davon (Rückwärtsprimer),
vorzugsweise ein Primerpaar von D und ein Primerpaar von E,
wobei die entsprechenden Derivate
a) zumindest 80% identisch zur jeweiligen oben aufgeführten Sequenz sind, oder
b) sich durch einen Basenaustausch, eine Insertion, Deletion oder Addition von einer jeweiligen Sequenz unterscheiden.

### Definitionen:

### Fragmente von Oligonukleotiden:

Fragmente von Oligonukleotiden entstehen durch Deletion von einem oder mehreren Nukleotiden am 5'- und/oder 3'- Ende eines Oligonukleotids.

### Gen:

Das Gen umfasst den offenen Leserahmen oder kodierenden Bereich einer DNA. Auch das Cistron ist ein Gen, das zusammen mit anderen Cistrons jedoch auf einer mRNA liegt. DNA-Regionen, die die Transkription des Gens regulieren, wie der Promotor, Terminator, Enhancer gehören ebenfalls zum Gen.

### Identische DNA-Sequenzen / Prozent Identität

Zur Bestimmung der Identität (im Sinne von vollständiger Übereinstimmung, entsprechend 100 % Identität) von DNA oder RNA-Sequenzen werden Teilsequenzen eines größeren Polynukleotids betrachtet. Diese Teilsequenzen umfassen 10 Nukleotide und sind dann identisch, wenn alle 10 Bausteine bei zwei Vergleichssequenzen identisch sind. Die Nukleotide Thymidin und Uridin seien identisch. Als Teilsequenzen können alle möglichen Fragmente eines größeren Polynukleotids betrachtet werden.

Als Beispiel seien zwei Polynukleotide betrachtet, die 20 Nukleotide umfassen und sich in dem 5. Baustein unterscheiden. In einem Sequenzvergleich findet man dann sechs 10-er Nukleotide, die identisch sind und 5, die nicht identisch sind, da sie sich in einem Baustein unterscheiden.

Außerdem kann die Identität graduell bestimmt werden, wobei die Einheit in Prozent angegeben wird. Zur Bestimmung des Grades der Identität werden auch Teilsequenzen betrachtet, die minimal die Länge der tatsächlich genutzten Sequenz, z.B. als Primer, oder aber 20 Nukleotide umfassen.

Als Beispiel werden Polynukleotide A mit einer Länge von 100 Nukleotiden und B mit eine Länge von 200 Nukleotiden verglichen. Aus Polynukleotid B wird ein Primer abgeleitet mit einer Länge von 14 Nukleotiden. Zur Bestimmung des Grades der Identität wird Polynukleotid A mit dem Primer in seiner ganzen Länge verglichen. Wenn die Sequenz des Primers in Polynukleotid A vorkommt, wobei sie aber in einem Baustein abweicht, dann gibt es ein Fragment mit einem ldentitätsgrad von 13:14 → 92,3 %.

Im zweiten Beispiel werden die zuvor genannten Polynukleotide A und B in ihrer Gesamtheit verglichen. In diesem Fall werden alle möglichen Vergleichsfenster einer Länge von 20 Nukleotiden angelegt und für sie der Identitätsgrad bestimmt. Sind also Nukleotid Nr. 50-69 von Polynukleotid A und B mit Ausnahme von Nukleotid Nr. 55 identisch, dann ergibt sich für diese Fragmente ein ldentitätsgrad von 19:20 → 95 %.

### Multiplex-PCR

Eine Multiplex-PCR sei eine Polymerase-Kettenreaktion oder DNA- oder RNA-Amplifikationsreaktion, in welcher mehr als zwei Primer verwendet werden, die nicht als Vorwärts-Rückwärts-Primerpaar angeshen werden. Dies führt bei Anwesenheit aller nachzuweisenden Nukleotidzielmoleküle zur Entstehung von mindestens zwei verschiedenen Amplikons. Diese Amplikons sollten sich zumindest in der Region, in welcher die Primer binden unterscheiden, sie können jedoch auch völlig unterschiedlichen Genen zuzuordnen sein. Im Falle des Nachweise der EHEC besteht die Multiplex-PCR im gleichzeitigen Nachweis von zwei oder drei Genen aus der Gruppe Sltl, Sltll, eae und hlyA.

### Nukleotide

Nukleotide sind die Bausteine der DNA oder RNA. Dabei bedeuten die Abkürzungen:
G = Guanosin, A = Adenosin, T = Thymidin, C = Cytidin, R = G oder A, Y = C oder T, K = G oder T, W = A oder T, S = C oder G, M = A oder C, B = C, G oder T, D = A, G oder T, H = A, C oder T, V = A, C oder G, N = A, C, G oder T, I = Inosin.

### Online-Detektion:

Online-Detektion ist im Zusammenhang mit der vorliegenden Erfindung definiert als der simultane Ablauf von zwei Vorgängen: der Detektion von DNA oder RNA und einem Prozess, der zur Bereitstellung einer detektierbaren Menge von DNA oder RNA führt. Bei diesem Prozess kann es sich z.B. handeln um die Freisetzung von genomischer DNA/RNA aus Zellen, um die Anreicherung von DNA/RNA aus einem komplexen Gemisch oder um die Amplifikation von Polynukleotiden z.B. durch eine PCR. Detektion sei die Wahrnehmung eines Signals, das mit dem Vorhandensein und eventuell der Menge von DNA/RNA korreliert. Im Falle der PCR kann ein solches Signal also mit zunehmender Amplifikation der Ziel-DNA steigen. Auch in miniaturisierter Form, z.B. auf einem Chip kann eine Online-Detektion durchgeführt werden. Das Signal kann z.B. durch fluoreszierende Moleküle einer Sonde, durch radioaktive Moleküle oder durch enzymgekoppelte Farb- oder Fluoreszenzintensivierung erzeugt werden.

Der Begriff Online-Detektion sei synonym zu Realtime-Detektion.

### Primer:

Primer sind Oligonukleotide, die als Startermoleküle bei einer PCR dienen. Dabei hybridisieren sie an ein Zielmolekül, bei welchem es sich z.B. um DNA oder RNA handeln kann, und werden durch eine Polymerase verlängert. Sie können aber gleichzeitig auch als Sonde dienen.

### Sonde:

Sonden sind Oligonukleotide, die an Ziel-DNA- oder RNA-Moleküle hybridisieren. Sie dienen der direkten oder indirekten Detektion dieser Ziel-DNA- oder RNA-Moleküle. Zu diesem Zweck können sie mit fluoreszierenden oder farbstoffhaltigen Molekülen gekoppelt sein. Außerdem können sie indirekt in einem ELISA nachgewiesen werden. In einer speziellen Ausführung erzeugen sie durch FRET (Fluoreszens-Resonanz-Energie-Transfer) nur dann ein Signal, wenn zwei Sonden in definierter Weise benachbart hybridisieren. In diesem Fall wird ein Farbstoff auf einer Sonde durch einen Lichtstrahl angeregt und überträgt seine Anregungsenergie auf den Farbstoff der benachbarten Sonde. Diese emittiert daraufhin Licht einer definierten Wellenlänge. Sie können gleichzeitig als Primer dienen.

### EHEC und VTEC

EHEC sind enterohämorhagische *E*. *coli* und eine Subgruppe der VTEC. *E*. *coli* des Serotyps O157 seien eine Subgruppe der EHEC.

VTEC zeichnen sich dadurch aus, dass sie entweder das Sltl (vtx1) oder das Sltll (vtx2) oder beide Gene besitzen. EHEC sind VTEC, die zusätzlich das eae-Gen und/oder hlyA-Gen (kodiert für Intimin) besitzen. Außerdem können sie durch das Vorhandensein weiterer Pathogenitätsgene, wie hlyB, hlyC, finA, fliC u.a. charakterisiert werden.

### Slt-Locus

Slt-Locus bedeutet den Locus enthaltend das Sltl-Gen bzw. Sltll-Gen, welche auch als vtxI-Gen bzw. vtxII-Gen bezeichnet werden. Die Nukleinsäuresequenz dieses Locus ist aus dem Stand der Technik bekannt, beispielsweise aus Paton, A.W. et al. 1995, Gene 153 (1), 71-74. Der Begriff "Locus", wie in diesem Zusammenhang verwendet, umfasst neben dem kodierenden Bereich weiterhin einen Abschnitt von jeweils 1000 Nukleotiden am 5'-Ende vom Startcodon bzw. am 3'-Ende vom Stopcodon.

### eae-Locus und hlyA-Locus

Die Sequenzen des eae-Locus bzw. hlyA-Locus sind ebenfalls aus dem Stand der Technik bekannt, wie beispielsweise aus Makino, K., et al. 1998, DNA Res. 5 (1), 1-9.

### Derivate der erfindungsgemäßen Oligonukleotide

Als Derivate der erfindungsgemäßen Oligonukleotide werden Sequenzen verstanden, die sich in mindestens einem Nukleotid von den spezifischen Sequenzen nach SEQ ID Nummern 1-98 unterscheiden, beispielsweise durch mindestens einen Basen-austausch, eine Insertion, Deletion oder Addition. Hierunter fallen auch Oligonukleotide, die zu mindestens 80 % identisch sind zu einer der spezifischen Sequenzen gemäß SEQ ID Nummern 1-98; sowie Oligonukleotide mit einer vergleichbaren Spezifität der Hybridisierung. Letzteres bedeutet, dass das Derivat das gleiche Hybridisierungsmuster mit einer vorgegebenen nukleinsäurehaltigen Probe ergibt, wie das Oligonukleotid mit einer der spezifischen Sequenzen mit einer der SEQ ID Nummern 1-98.

Die zum EHEC-Nachweis entsprechend der vorliegenden Erfindung essentiellen sequenzen sind in den Ausfführungsformen I und II angegeben.

### Biochip

Unter Biochip werden Träger für den Hochdurchsatz an Analysen verstanden, wie sie beispielsweise von AFFYMETRIX vertrieben werden. Die Chips ermöglichen das Austesten von zahlreichen verschiedenen Nukleinsäuren auf einem Trägers.

Die Analytik von DNA weist wesentliche Vorteile gegenüber dem serologischen Nachweis auf, da es für die DNA-Analytik standartisierte, einfache Reinigungsverfahren gibt, mit denen DNA von externen Matrizes getrennt und aufgereinigt werden kann. Auf Grund der Größe des bakteriellen Genoms kann man außerdem aus einer beträchtlichen Zahl von individuellen Sequenzmotiven auswählen, während die Auswahl der zuvor erwähnten exponierten Oberflächenantigene relativ gering ist.

Als Sequenzen zum spezifischen Nachweis von EHEC-Bakterien eignen sich Sequenzen aus dem Slt-Locus, dem eae-Locus und dem hlyA-Locus. Dabei reicht es für den Nachweis von EHEC in einer vorgegebenen Probe aus, wenn eine Teilsequenz aus dem Slt-Locus und einem weiteren der genannten Loci in der Analysenprobe nachgewiesen werden kann.. Bei dem Slt-Locus handelt es sich tatsächlich um zwei verschiedene Genloci, Sltl und Sltll, wobei jedoch bei zahlreichen EHEC-Stämmen nur einer der beiden Loci vorkommt Eine ausreichend hohe Aussagekraft besitzt der gleichzeitige Nachweis von Sequenzen aus dem Slt-Locus und dem eae-Locus in einer einzigen Probe. Eine ähnlich hohe Zuverlässigkeit besitzt der gleichzeitige Nachweis einer Sequenz aus dem Slt-Locus und dem hlyA-Locus. Ein besonders hoher Grad an Zuverlässigkeit hinsichtlich einer EHEC-Kontamination ist dann gegeben, wenn Sequenzen aus den drei verschiedenen Loci Slt, eae und hlyA gleichzeitig in einer Probe nachgewiesen werden.

Bei einer weiteren bevorzugten Ausführungsform wird die zu untersuchende Nukleinsäure einer PCR zugeführt. Diese hat zur Folge, dass EHEC-spezfische Amplicons erzeugt werden, sofern Nukleinsäuren von EHEC-Bakterien in der Probe vorliegen. Dabei lässt sich im einfachsten Fall die PCR als einfache lineare PCR mit nur einem Oligonukleotid als Primer ausgestalten, vorzugsweise erfolgt die PCR jedoch mit sog. Vorwärts- und Rückwärtsprimern für jeden zu amplifizierenden Genomabschnitt der bakteriellen Nukleinsäure.

Seq ID Nrn 1-45 und 95-98 werden Kategorien A-C zugeordnet, Seq ID Nrn 46-83 und 93-94 werden Kategorien D-E zugeordnet.

Die erfindungsgemäßen Ausfführungsformen I und II umfassen die oben angegebenen Sequenzen aus den Kategorien A bis E.

Bei einer weiteren bevorzugten Ausführungsform umfasst der Nachweis den Einsatz eines weiteren Primers umfassend mindestens eine Sequenz, ausgewählt aus einer Sequenz der Kategorie F. Diese Sequenzen sind charakteristisch für die Gattung *E*. *coli.* So kann beispielsweise bei einer bevorzugten Strategie des EHEC-Nachweises die Analysenprobe zunächst mit einer Sequenz, ausgewählt aus der Kategorie F analysiert werden. Ein positives Ergebnis deutet auf das Vorliegen von *E. coli* in der Analysenprobe hin. In einem zweiten Schritt lässt sich dann näher spezifizieren unter Einsatz der Sequenzen aus den Kategorien A - E, ob es sich bei dem nachgewiesenem *E. coli* um ein Mitglied der EHEC-Gruppe handelt. Die zusätzliche Analyse mit Sequenzen aus der Kategorie F könnten natürlich auch als zusätzliche Maßnahme nach der Analyse mit den Sequenzen aus den Kategorien A - E erfolgen.

Bei einer weiteren bevorzugten Ausführungsform werden die verschiedenen Oligonukleotide und somit die verschiedenen PCR-Ansätze in Form einer Multiplex-PCR durchgeführt. Dabei werden in einem einzigen Reaktionsansatz mit Hilfe der verschiedenen Oligonukleotide verschiedene Amplikons in der PCR erzeugt. Alternativ lässt sich die Mulitplex-PCR auch aufteilen auf verschiedene PCRs, wobei eine sequenzielle Abfolge von PCR durchgeführt wird, wobei jede PCR mit einem spezifischen Primer bzw. Primerpaar durchgeführt wird. In beiden Fällen wird bei Vorliegen von EHEC-Bakterien in der Analysenprobe ein Bandenmuster erhalten, das das Vorhandensein von EHEC-Bakterien anzeigt

Bei einer weiteren bevorzugten Ausführungsform wird in dem Nachweisverfahren von der sog. Chip-Technologie (Biochips) Gebrauch gemacht Dabei lässt sich einerseits eine Vielzahl an verschiedenen Analysenproben auf einem Chip analysieren, indem die einzelnen Spots auf dem Chip-Analysenmaterial aus verschiedenen Quellen enthalten. Andererseits kann der Chip einen Satz Oligonukleotide tragen, wobei jeder Spot ein spezifisches Oligonukleotid enthält und dieses Origonukleotidmuster mit Analysenproben in Kontakt gebracht wird. Falls das Analysenmaterial EHEC-Nukleinsäure enthält, hybridisiert diese mit den auf den auf dem Chip vorgelegten EHEC-spezfischen Sonden und ergibt ein entsprechendes Signalmuster.

Bei einer weiteren bevorzugten Ausführungsform kann der Nachweis weitere Schritte umfassen, wie z. B. eine Amplifikation der nachzuweisenden Nukleinsäure, wobei diese vorzugsweise mittels PCR erfolgt und/oder eine Southemhybridisierung mit EHEC-spezifischen Sonden, wobei diese Hybridisierung ohne vorherige Amplifikation oder nach erfolgter Amplifikation der nachzuweisenden Nukleinsäure erfolgt. Weiterhin lässt sich die nachzuweisende Nukleinsäure mittels der Ligase-kettenreaktion nachweisen. Schließlich kann die nachzuweisende Nukleinsäure mittels isothermer Nukleinsäureamplifikation angereichert werden.

Bei einer weiteren bevorzugten Ausführungsform lässt sich das Amplifizieren der Zielnukleinsäure auch mittels einer Online-Detektion verfolgen.

Bei einer weiteren bevorzugten Ausführungsform erfolgt die Amplifikation der nachzuweisenden Nukleinsäure und/oder die Detektion der erhaltenen Amplikons auf einem Biochip, wobei es besonders bevorzugt ist, die Amplifikation und Detektion auf einem Chip durchzuführen.

Erfindungsgemäße Mittel zum Durchführen des oben beschriebenen Verfahrens von Ausführungsform I sind Oligonukleotide von Ausführungsform II.

Die Oligonikleotide lassen sich einerseits als Primer im Rahmen einer PCR einsetzen und andererseits auch als Sonden, beispielsweise im Rahmen einer Southemblothybridisierung. Je nach den Bedürfnissen des gewünschten Nachweises kann der Fachmann die geeignete Kombination an Oligonukleotiden als Primer bzw. Sonden zusammenstellen.

Bei einer weiteren besonders bevorzugten Ausführungsform umfasst die erfindungsgemäße Kombination weiterhin ein Oligonukleotid ausgewählt aus Sequenzen der Kategorie F, welche spezifisch für die Gattung *E*. *coli* sind. Vorzugsweise kommen die genannten Oligonukleotide bzw. Kombinationen davon in Form eines Kits zum Nachweis von EHEC-Bakterien zum Einsatz, wobei der Kit auch weitere Reagenzien zum Nachweis der Bakterien bzw. zum Durchführen der Nachweisreaktionen umfasst Hierzu zählen beispielsweise die für die PCR erforderlichen Reagenzien und Enzyme und ggf. geeignete Trägermaterialen, wie beispielsweise bei der Chip-Technologie gewünscht

Die erfindungsgemäßen Oligonukleotide bzw. Oligonukleotidkombinationen sind somit ein geeignetes Mittel zum spezifischen und sicheren Nachweis von EHEC-Bakterien in beliebigen Analysenproben.

Mit der Erfindung der Polymerasekettenreaktion ist es möglich einzelne DNA-Polynukleotide zu amplifizieren, und anschliessend mit extrem hoher Empfindlichkeit nachzuweisen. Diese Technik eröffnet große neue Möglichkeiten, birgt in sich jedoch auch neue Probleme. So können bei der DNA-Amplifikation leicht falsche Fragmente amplifiziert werden, die zu falsch-positiven Ergebnissen in der Analytik führen. Außerdem ist es sehr schwierig aus der Fülle der Möglichkeiten die diagnostischen DNA-Sequenzen auszuwählen, die charakteristisch für EHEC sind.

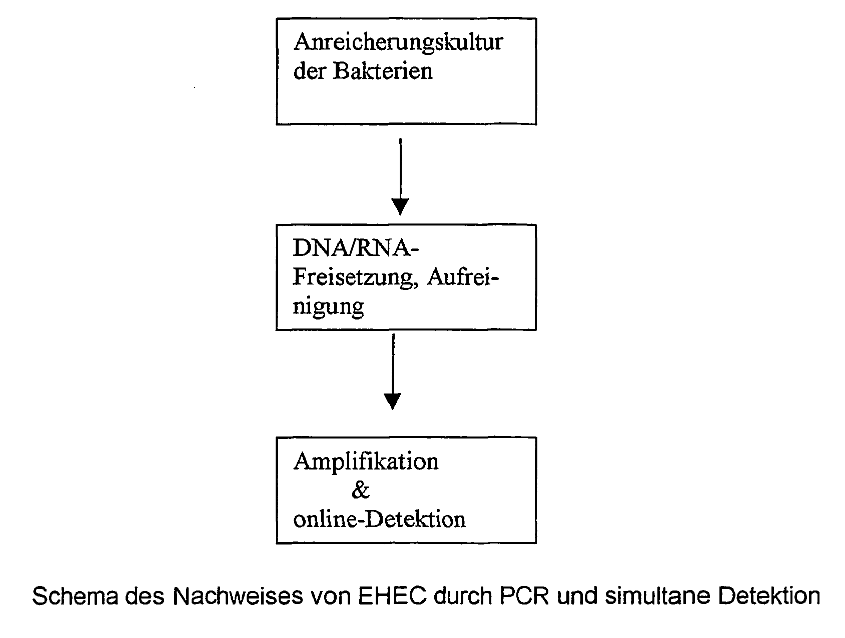

Die vorliegende Erfindung besteht in Verfahren und Oligonukleotiden, die einen qualitativen und quantitativen Nachweis der EHEC erlauben. Dieses Verfahren beinhaltet auch eine Positivkontrolle für die PCR-Reaktion, die die Gattungen *E*. *coli* und Shigella nachweist. Dies ist wichtig, da bei negativen EHEC-Befunden der korrekte Ablauf der PCR-Reaktion sichergestellt sein muss. Das Nachweisverfahren besteht im Ganzen aus vier Schritten: Vermehrung der Bakterien, Aufreinigung der DNA/RNA, Amplifikation der Polynukleotide und Detektion derselben. In einem besonderen Verfahren können die beiden letzteren Schritte auch simultan erfolgen.

Die Vermehrung der Bakterien erfolgt indem die zu untersuchende Matrix, z.B. ein Lebensmittel oder Fäkalproben, mit einem gängigen bakteriellen Medium inkubiert werden. Bakterielle Medien sind kommerziell erhältlich und können z.B. eine proteolytisch verdaute Grundsubstanz, wie Sojabouillon, Gallensalze, und einen Puffer, wie Dikaliumhydrogenphosphat, enthalten. Außerdem ist es vorteilhaft dem Anreicherungsmedium einen Inhibitor zuzusetzen, welcher das Wachstum der EHEC gegenüber anderen Bakterien im Anreicherungsmedium begünstigt. Solche Inhibitoren können Antibiotika, wie z.B. Novobiocin sein.

Im zweiten Schritt werden die Polynukleotide aufgereinigt. Hierzu werden die Bakterien i.d.R. zunächst durch Zentrifugation und/oder Filtration vom Medium getrennt. Es kann ein weiterer Waschschritt erfolgen. Anschließend werden die Bakterien aufgeschlossen. Dies erfolgt durch Erhitzen, durch ein alkalisches oder saures Milieu oder durch Reagenzien, die die Bakterienzellwand destabilisieren, wie deionisierende Chemikalien oder Lysozym. Die genomische DNA bzw. die RNA kann nun direkt in eine PCR-Reaktion eingesetzt werden, oder sie wird weiter aufgereinigt. Für diese Aufreinigung geeignet sind Materialien, an deren Oberfläche die Polynukleotide binden, wie z.B. positiv geladene Oberflächen oder Silikat-Oberflächen. Dieses Material kann in Säulen eingebaut sein und ist kommerziell erhältlich.

Die größte Bedeutung beim Nachweis der Bakterien kommt der PCR-Reaktion und der Detektion der Amplikons zu. Wie bereits zuvor erläutert ist es sehr schwierig, Unterschiede in DNA-Sequenzen zwischen EHEC und anderen Bakterien, insbesondere harmlosen *E. coli*-Stämmen zu finden. Eine singuläre PCR-Reaktion mit der Amplifikation eines einzigen DNA- oder RNA-Bereiches alleine scheint kein sehr sicheres Fundament der Stammabgrenzung zu bilden. Ein bevorzugtes Element der Erfindung besteht darin, das verschiedene Bereiche des EHEC-Genoms simultan und/oder sequenziell amplifiziert werden. Vorzugsweise- werden zur abschließenden Analyse weitere DNA/RNA-Sequenzen dann in einem konsekutiven Schritt amplifiziert. Wenn alle signifikanten Amplikons simultan detektiert werden können, z.B. auf einem Chip, dann können der "erste" Amplifikationsschritt und der "konsekutive" Amplifikationsschritt auch in einer einzigen PCR-Reaktion, bzw. in einem einzigen PCR-Reaktionsgefäß ablaufen. Der Schlüssel zur Verwendung der Primer und Sonden ist nachfolgend gegeben.

Das System zur Detektion von EHEC stellt Primer zur Verfügung, die In bestimmten Kombinationen die Gruppe der EHEC optimal abbilden. Der Nachweis wird z. B. in zwei unabhängigen PCR-Läufen in Primer-Multiplexansätzen durchgeführt. In einem ersten Lauf werden Primer und Sonden der Kategorie A, B und/oder C entsprechend Ausfführungform I oder II eingesetzt In dem zweiten Lauf werden nur Proben verwendet, die im ersten Lauf positiv waren. In diesem zweiten Lauf werden Primer und Sonden der Kategorien D und E entsprechend Ausfführungsform I oder II verwendet. Innerhalb einer Kategorie sind jeweils ein Vorwärtsprimer und ein Rückwärtsprimer miteinander kombinierbar. Es werden also Multiplex-PCRs durchgeführt, in denen in einer Reaktion gleichzeitig mehrere Ziel-DNA- oder -RNA-Fragmente vermehrt werden. Auf Grund dieses Vorgehens lässt sich ein sehr differenziertes Bild von der vorliegenden bakteriellen Population gewinnen. Je nach den praktischen Anforderungen an die Sensitivität des EHEC-Nachweises und wenn die simultane Detektion möglich ist, können auch alle Nachweisprimer (für Kategorie A+B+C und D oder E und eventuell Kategorie F) in einer einzigen Multiplex-PCR eingesetzt werden. Die erfindungsgemäß essentiellen Sequenzkombinationen sind in den Ausfführungsformen I und II angegeben.

**Tab.1: Vorwärtsprimer Kategorie A**

| Nr. | Sequenz des Primers |
|---|---|
| 1 | CTGGGGAAGGTTGAGTAG |
| 2 | GTCCTGCCTGAYTATCATGG |
| 3 | ACAAGACTCTGTTCGTGTAGG |
| 4 | AAGAATTTCTTTTGRAAGYRTTAATGC |
| 5 | AATTCTGGGWAGCGTGGCATTAATACTG |

**Tab.2: Rückwärtsprimer Kategorie A**

| Nr. | Sequenz des Primers |
|---|---|
| 6 | CCCACTTTAACTGTAAAGGT |
| 7 | CGTCATCATTATATTTTGTATACTCCACC |
| 8 | CACTTGCTGAAAAAAATGAAAG |

**Tab.3: Sonden Kategorie A**

| Nr. | Sequenz der Sonde | Sondenpaar |
|---|---|---|
| 9 | AGCGTGGCATTAATACTGAATTGTCA | 1 |
| 10 | ATCATGCATCGCGAGTTGCCAGAAT | 1 |
| 11 | GTCCTGCCTGAMTATCATGGACAAGACTCT | 2 |
| 12 | TTCGTGTWGGAAGAATTTCTTTTGRAAGYRTTAAT | 2 |
| 13 | ATGAGTTTCCTTCTATGTGYCCGGYAGATGGAA | 3 |
| 14 | TCCGTGGGATTACGCACAATAAAATATTTGTGGGATT | 3 |
| 15 | AAAYATTATTAATAGCTGCATCRCTTTCATTT | 4 |
| 16 | TTCAGCAAGTGYGCTGGCKRCGCCWGATTCTGTA | 4,5 |
| 17 | ACTGGRAAGGTGGAGTATACAAAATATAATGAT | 5 |
| 95 | ATTAAYRCTTYCAAAAGAAATTCTTCC | 6 |
| 96 | CAGTATTAATGCCACGCTWCCCAGAATT | 6 |
| 97 | CCTTCTATGTGYCCGGYAGATGGAA | 7 |
| 98 | TSCGTGGGATTACGCACAAT | 7 |

**Tab.4: Vorwärtsprimer Kategorie B**

| Nr. | Sequenz des Primers |
|---|---|
| 18 | GGCACTGTCTGAAACTGCT |
| 19 | GAAACTGCTCCTGTKTATAC |
| 20 | GATGACRCCGGRAGAMGTG |
| 21 | CTGAACTGGGGGMGAATCAGCAATGTG |

**Tab.5: Rückwärtsprimer Kategorie B**

| Nr. | Sequenz des Primers |
|---|---|
| 22 | YGCCATTGCATTAACAGA |
| 23 | GCWGCKGTATTACTTTCCCATAA |
| 24 | GGCCTGTCGCCAGTTATCTGACATTCTGGTTG |
| 25 | TCTCTTCATTCACGGCGCG |

**Tab.6: Kategorie C Vorwärtsprimer**

| Nr. | Sequenz des Primers |
|---|---|
| 26 | GGCGCTGTCTGAGGCATCT |
| 27 | GAGGCATCTCCGCTTTATAC |
| 28 | AATGACGGCTCAGGATGTT |
| 29 | CTGAACTGGGGAAGAATAAGTAATGTT |

**Tab.7: Kategorie C Rückwärtsprimer**

| Nr. | Sequenz des Primers |
|---|---|
| 30 | GCAGCGATTGTATTCGCTTCCCACAAAACA |
| 31 | GCCCTGTCTCCAACAATCTGGCATTCTGTTTT |
| 32 | CTGTTTTTGGCTCACGGAACG |
| 33 | CGCCATGGAATTAGCAGAAAAG |

**Tab.8: Sonden Kategorie B**

| Nr. | Sequenz der Sonde | Sondenpaar |
|---|---|---|
| 34 | CCCCAGTTCAGWGTGAGGTCC | 1 |
| 35 | CCGGAAGCACATTGCTGATTC | 1 |
| 36 | GAATATCCTTTAATAATATATCAGCGATACTKGG | 2 |
| 37 | WGTGGCSGTTATACTGAATTGYCATCATCAGGG | 2 |
| 38 | CGTTCYGTTCGCKCCGTGAATGAAGAKA | 3 |
| 39 | CAACCAGAATGTCAGATAACTGGCGACAGGCC | 3 |

**Tab.9: Sonden Kategorie C**

| Nr. | Sequenz der Sonde | Sondenpaar |
|---|---|---|
| 40 | CCCCAGTTCAGGGTAAGGTCA | 1 |
| 41 | CTGGAAGAACATTACTTATTC | 1 |
| 42 | AGGATATCTTTTAATAGTCTTTCTGCGATTCTCGG | 2 |
| 43 | TGTTGCGGTCATCCTTAATTGCCACTCAACCGG | 2 |
| 44 | TTATTCAGTTCGTTCCGTGAGCCAAAAAC | 3 |
| 45 | AAAACAGAATGCCAGATTGTTGGAGACAGGGC | 3 |

**Tab.10: Kategorie D Vorwärtsprimer**

| Nr. | Sequenz des Primers |
|---|---|
| 46 | CATGCTGCITTTTTAGAAGA |
| 47 | CATGCTGCRTTTTTAGAAGA |
| 48 | CATGCTGCITTTTTAGAAGACTCT |
| 49 | CATGCTGCRTTTTTAGAAGACTCT |
| 50 | AATGAATGGGAAAAGGAGCATGGC |
| 51 | CTCTCTGTCTTTGCTTGCTGATT |
| 52 | CTCGTCAGCATGCAGTAGAAAGAGCAGTCG |
| 53 | CATTGGGATGAGAAGATCGGTGAACTTGCAGG |

**Tab.11: Kategorie D Rückwärtsprimer**

| Nr. | Sequenz des Primers |
|---|---|
| 54 | CGTCTTTATCTCCGAGYTCAG |
| 55 | ACATCGTCTTTATCTCCGAGYTCAG |
| 56 | TTTACCAACATCCGTCTTATTATAAGATACGG |
| 57 | CCTTCACCAGCAAATACTTCTG |
| 58 | TGAGCCTGCTCCAGAATAAACC |
| 59 | TCAATTTTGAATAATCATATACA |

**Tab.12: Sonden Kategorie D**

| Nr. | Sequenz der Sonde | Sondenpaar |
|---|---|---|
| 60 | AGAGAAAGAAAACAGAGTGGTAAATATGAATATATGACAT | 1 |
| 61 | TCTTATTGTAAATGGTAAGGATACATGGTCTGTAAAAG | 1 |
| 62 | GGGACCATAGACCTTTCAACAGGTAATGTATCAAGTGTTTT | 2 |
| 63 | ACATTTATAACACCAACATTTACCCCAGGAGAAGAAG | 2 |
| 64 | GGCATATATTAATTATCTGGAAAATGGAGGGCTTTTAGAGGC | 3 |
| 65 | CAACCGAAGGAGTTTACACAACAAGTGTTTGATCCTC | 3 |
| 66 | CATTGGGATGAGAAGATCGGTGAACTTGCAGGCAT | 4 |
| 67 | AACCCGTAATGCTGATCGCAGTCAGAGTGGTAAGGC | 4 |

**Tab.13: Kategorie E Vorwärtsprimer**

| Nr. | Sequenz des Primers |
|---|---|
| 68 | GGCCTGGTTACAACATTATGG |
| 69 | ACGCGAAAGATACCGCTCTTGGTAT |
| 70 | CCAGGCTTCGTCACAGTTGCA |
| 71 | GGAACGGCAGAGGTTAATCTGCAG |
| 72 | AGTGGTAATAACTTTGACGGTAGTTC |

**Tab.14: Kategorie E Rückwärtsprimer**

| Nr. | Sequenz des Primers |
|---|---|
| 73 | ATCCCCATCGTCACCAGA |
| 74 | AACATTATCACCATAATACTG |
| 75 | TAGTTTACACCAACGGTCGCCGC |
| 76 | CATTACCCGTACCATGACGGT |
| 77 | CGGAACTGCATTGAGTAAAGGAGATCA |

**Tab.15: Sonden Kategorie E**

| Nr. | Sequenz der Sonde | Sondenpaar |
|---|---|---|
| 78 | TCCAGTGAACTACCGTCAAAGTTATYACCAC | 1 |
| 79 | CCAGCATKTTTTCGGAATCATAGAACGGTAATAAGAA | 1 |
| 80 | ATGTTGGGCTATAACGTCTTCATTGATC | 2 |
| 81 | AGGATTTTTCTGGTGATAATACCCGT | 2 |
| 82 | AGGTATTGGTGGCGAATACTGGCGAGACTATTTCAAAAGTAG | 3 |
| 83 | TTAACGGCTATTTCCGCATGAGCGGCTGGCATGAGTCATAC | 3 |
| 93 | TCCAGTGAACTACCGTCAAAGTTATYACCAC | 4 |
| 94 | CCAGCATKTTTTCGGAATCATAGAACGGTAATAAGAA | 4 |

Zusätzlich zum Nachweis der EHEC ist es vorteilhaft den korrekten Ablauf des Verfahrens zu kontrollieren. Die vorliegende Erfindung gewährleistet diese Kontrolle, indem sie den gattungsspezifischen Nachweis von *E. coli* ermöglicht. So ist insbesondere eine Differenzierung gegenüber Enterobakterien, wie der Gattung Citrobacter, vorteilhaft, da diese Bakterien in vielen Fällen in einem horizontalen Gentransfer Pathogenitätsgene von *E. coli* übernommen haben. Eine falschpositive Klassifizierung als VTEC kann somit vermieden werden. Da *E. coli* und Shigella in molekularbiologischer Betrachtungsweise und auch in vielen taxonomischen Einordnungen eine Einheit bilden, werden diese beiden Gattungen bei der Kontrolle nicht getrennt. Dies ist in der Praxis sehr sinnvoll, da in der mikrbiologischen Routinediagnostik in der Regel auch nicht mehr zwischen diesen Gattungen differenziert wird.

Tab. 16+17 enthält Primer, die den Nachweis von *E*. *coli* und Shigella erlauben. Für die Untersuchung können Aliquots der gleichen DNA/RNA-Proben verwendet werden wie für den EHEC-Nachweis. Außerdem ist es möglich die *E*. *coli-*Kontrollreaktion simultan, d.h. in einem Reaktionsgefäß zusammen mit dem EHEC-Nachweis, oder parallel durchzuführen. Des weiteren ist der *E. coli*/Shigella-Nachweis auch geeignet diese Gattungen von anderen zu unterscheiden.

**Tab.16: Kategorie F Vorwärtsprimer**

| Nr. | Sequenz des Primers |
|---|---|
| 84 | CGG GTC AGG TAA TTG CAC AGT A |
| 85 | CGG GTC AGG TGA TTG CAC AGT A |
| 86 | CGG GTC AGG TGA TTG CAC AAT A |
| 87 | CGG GTC AGG TAA TTG CAC AAT A |

**Tab.17: Kategorie F Rückwärtsprimer**

| Nr. | Sequenz des Primers |
|---|---|
| 88 | GCA ACA GTT CAG CAA AGT CCA T |

**Tab.18: Sonden Kategorie F**

| Nr. | Sequenz der Sonde | Sondenpaar |
|---|---|---|
| 89 | CGG TGA AGC CAC CGA CAT CGT | 1 |
| 90 | TGG CAG GTT CCG GCC TTC ACT CTC | 1 |
| 91 | AAGCCACCGACATCGTG | 2 |
| 92 | AAGCCACTGACATCGTG | 2 |

Der Nachweis der Amplikons kann durch Gelelektrophorese und Detektion der DNA-Banden erfolgen. Alternativ können die Amplikons mit Hilfe von Sonden detektiert und auch quantifiziert werden. Es gibt verschiedene Möglichkeiten die Sonden so zu modifizieren, dass eine direkte oder indirekte Visualisierung ermöglicht wird. So können sie mit einem Ankermolekül gekoppelt werden, das als Linker dient. Ein solches Ankermolekül kann z.B. ein Protein sein, dass von einem Antikörper erkannt wird. Dieser Antikörper kann mit einem Enzym gekoppelt sein, dass eine Farbreaktion hervorruft, wodurch die Detektion zustande kommt. z.B. wird die Peroxidase oder Katalase für diese Zwecke eingesetzt. Außerdem kann eine Sonde auch radioaktiv markiert werden, wobei die Messung der radioaktiven Aktivität zum Nachweis und zur Quantifizierung führt.

Eine weitere Möglichkeit besteht darin, ein fluoreszierendes Molekül an die Sonde zu koppeln. In diesem Fall muss gewährleistet sein, dass die Fluoreszenz nur emittiert oder detektiert wird, wenn die Sonde an einen Einzelstrang des Amplikons bindet. Dies kann erreicht werden, indem das Sonden-Amplikon-Hybrid von dem übrigen PCR-Gemisch getrennt wird. Z.B. können Sonden an feste Oberflächen gebunden werden, die Einzelstrangamplikons "einfangen", wobei freie Sonden abgewaschen werden.

Eine elegante Methode besteht in der Online-Detektion der PCR-Produkte. In diesem Fall kommt es nur dann zu einem Fluoreszenssignal, wenn eine fluoreszenzmarkierte Sonde an ein Amplikon anlagert. Dieses kann geschehen, indem der Sondenanteil des Amplikon-Sonden-Hybrids selektiv enzymatisch abgebaut wird. Auch kann durch die Entfaltung der Sonde, wenn sie sich an das Amplikon bindet, ein Quenching des Fluoreszenzsignals aufgehoben werden.

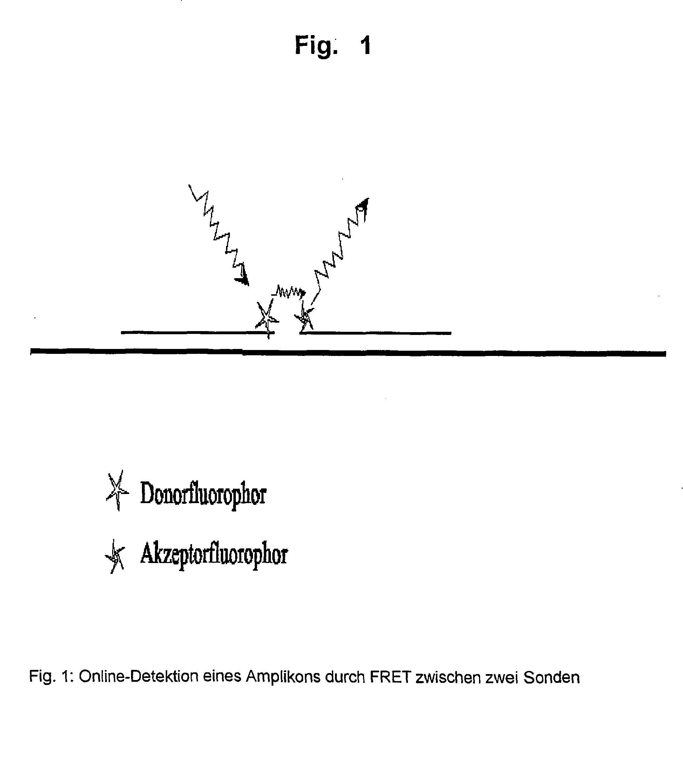

Eine weitere Möglichkeit ist, dass zwei fluoreszenzmarkierte Sonden eingesetzt werden. Nur wenn beide benachbart an ein Amplikon binden, kann ein sogenannter FRET (Fluoreszens-Resonanz-Energie-Transfer) ein Signal erzeugen (Fig. 1). Dieses Verfahren hat den großen Vorteil, dass mehrere Spezifitätsebenen Bestandteil des Nachweises sind: erstens binden die Primer an ein bestimmtes Zielmolekül, zweitens müssen beide Sonden an das "richtige" Amplikon binden und drittens dabei in der richtigen Reihenfolge nebeneinander liegen. Bei dieser benachbarten Anordnung ist außerdem der Abstand der Sonden für die erfolgreiche Emission des Signals entscheidend. Jedes dieser Erfordernisse leistet einen Betrag zum Erhöhen der Spezifität des Nachweises.

Alternativ gibt es auch Fluoreszenzmoleküle, die in die DNA-Doppelhelix interkalieren und dann ein Signal emittieren. Dieser unspezifische Nachweis von PCR-Produkten hat jedoch den Nachteil, dass fehlerhafte Amplifikationsprodukte auch detektiert werden.

Die Durchführung der Untersuchung bedarf nach der obigen Beschreibung einer großen Zahl von Komponenten. Deshalb ist es besonders vorteilhaft diese in einer oder mehreren Verpackungseinheiten eines Kits anzubieten. Ein solches Kit kann also die Reagenzien und Chemikalien für die Bakterienanreicherung, die Komponenten für die DNA-Freisetzung und Reinigung, wie auch das Verbrauchsmaterial zur Durchführung der PCR und zur Detektion enthalten.

### Figurenbeschreibung

Figur 1 zeigt chematisch das Prinzip des FRET
Figur 2 zeigt PCR-Produkte mit Primern der Kategorie D
Figur 3 zeigt PCR-Produkte mit Primern der Kategorie E
Figur 4 zeigt die Amplifikation und Realtime-Detektion der SltI- und SltII-Gene bei EHEC-Stämmen.
Figur 5 zeigt die Amplifikation und Realtime-Detektion der eae-Gene bei EHEC-Stämmen in einer Multiplex-PCR-Reaktion zusammen mit den Slt-Genen.

### Die folgenden Beispiele erläutern die Erfindung

Die gezeigten Figuren 1 - 5 wurden unter folgenden Bedingungen erstellt:
- Figur 1:: Es ist der schematische Ablauf des FRET gezeigt. Es stehen zahlreiche Kombinationen an Donor und Akzeptor zur Verfügung. Wichtig ist jedoch, dass sich das Absorptionsspektrum des Akzeptors mit dem Emissionsspektrum des Donors überlappt. Nur so ist sichergestellt, dass eine Anregung des Donors auch zu einer ausreichend starken Floureszenz beim Akzeptor führt.

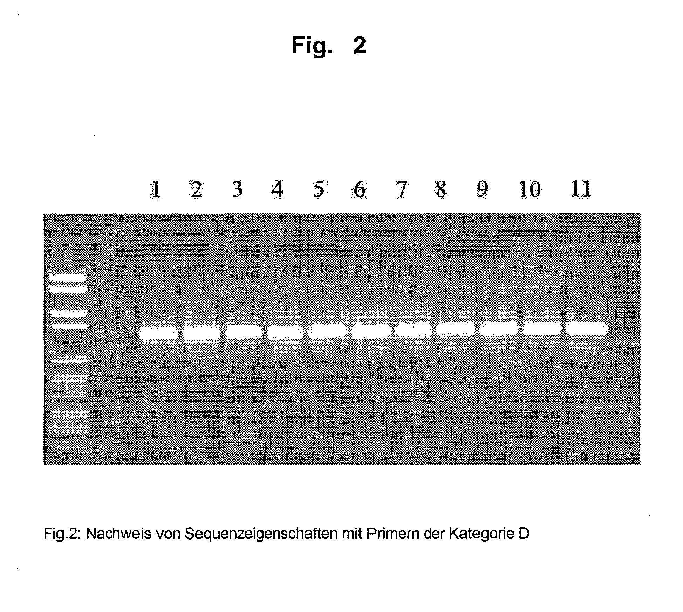

- Figur 2:: Nachweis von EHEC mit Primern der Kategorie D. Die Versuchsbedingungen entsprechen im Wesentlichen den im Kapitel "Nachweis von EHEC-Stämmen durch PCR". Der Nachweis im Agarosegel erfolgte ebenfalls wie im obigen Kapitel beschrieben.

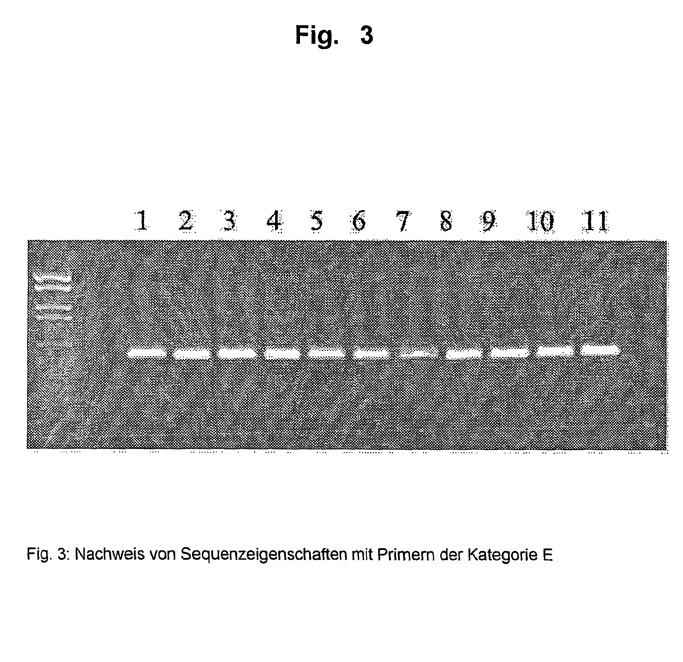

- Figur 3:: Nachweis von EHEC mit Primern der Kategorie E. Die Versuchsbedingungen entsprechen im Wesentlichen den im Kapitel "Nachweis von EHEC-Stämmen durch PCR". Der Nachweis im Agarosegel erfolgte ebenfalls wie im obigen Kapitel beschrieben.

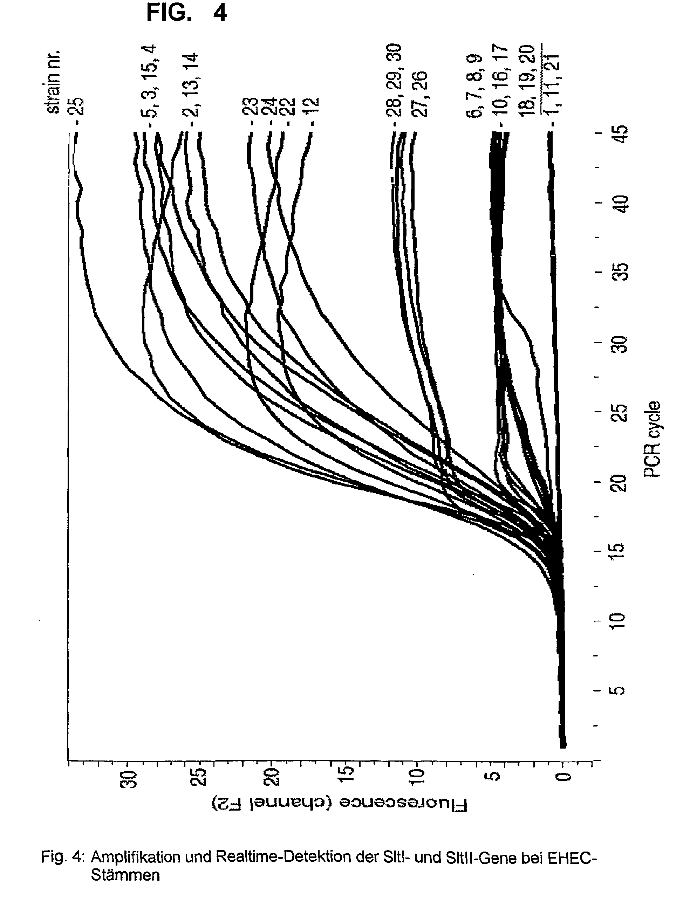

- Figur 4:: Diese zeigt die Amplifikation von Sltl- und Sltll-Genen durch RealtimePCR. Verwendet wurden Sonden, die die Detektion sowohl der Sltl- als auch der Sltll-Gene erlauben. Diese wurden jeweils mit gleichen Fluoreszenzfarbstoffen (Lighcycler RED 640 und Fluorescein) gekoppelt, so dass die Detektion in nur einem Kanal (F2) erfolgt. Es ist zu erkennen, dass bei der Amplifikation der SltII-Gene Signalkurven mit Amplituden entstehen, die größer als 14 sind. Die Signalkurven der SltI-Gene liegen signifikant tiefer. Wenn sowohl SltI- als auch SltII-Gene vorkommen, dann weist die Amplitude das höchste Niveau auf. Sie ist somit als Indikator für das Vorkommen und die Differenzierung zwischen den SltI- und SltII-Genen geeignet.
Aus Figur 4 ist auch zu sehen, dass je nach Verwendung verschiedener Sonden die Signalamplitude für die SltI-Gene unterschiedlich hoch ist. Für das abgebildetet Experiment wurden die Primer Nr. 1+6 und Nr. 18+22, sowie die Sonden Nr. 9+10 (für Stamm-Nr. 1-10), Sonden Nr. 95+96 (für Stamm-Nr. 11-20), Sonden Nr. 97+98 (für Stamm-Nr. 21-30) und Sonden 34+35 (für Stamm Nr. 1-30) verwendet. Die Sonden waren mit den Farbstoffen Fluorescein und Lightcycler Red640 gekoppelt. Die Detektion erfolgte bei einer Lichtwellenlänge von 640 nm.
Es ist zu erkennen, daß die Sonden Nr. 97+98 bei Stämmen, die nur das SIt1-Gen (s. Tabelle S. 56) besitzen, die höchste Amplitude ergeben. Diese Sonden-Primerkombination ist für die online-PCR deshalb besonders gut geeignet..
Nachweis der Slt-Gene: 25 (Slt2 ohne eae)// 5, 15 (Slt2 ohne eae), 3, 4 (Slt2+eae)// 2 (Slt2 ohne eae) 13, 14 (Slt2+eae)//23 (Slt2+eae)//24 (Slt2+eae)//22 (Slt2 ohne eae)// 12 (Slt2 ohne eae)//28, 29, 30 (Slt1+eae), 27 (Slt1+eae), 26 (Slt1+Slt2+eae)//6, 16 (Slt1+Slt2+eae), 7, 8, 9, 10, 17, 18, 19, 20 (Slt1+eae)//1, 11, 21 (Wasser).

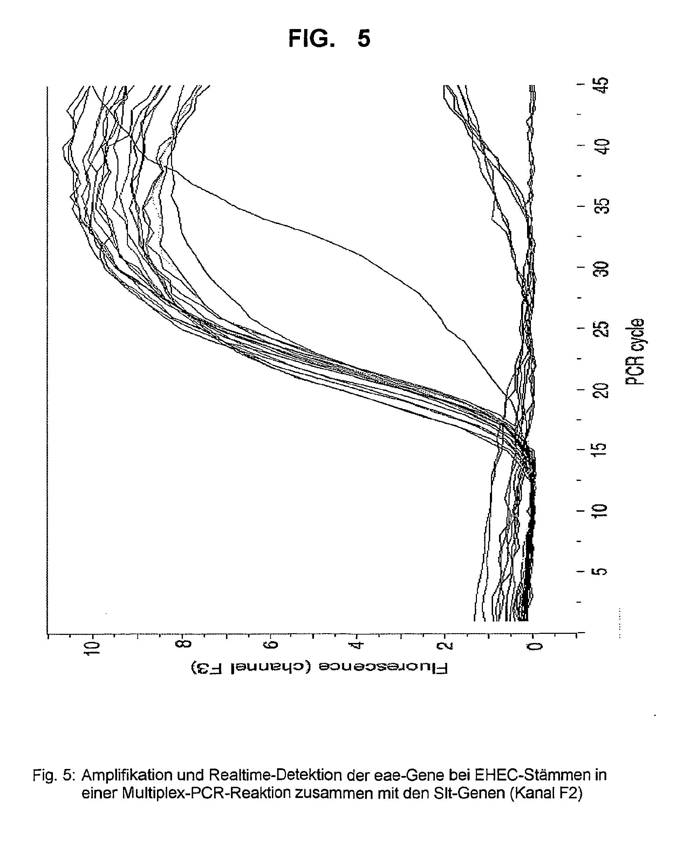

- Figur 5:: Sie zeigt die Amplifikation und Realtime-Detektion der eae-Gene bei EHEC-Stämmen in einer Multiplex-PCR mit den Slt-Genen (Fig.4).
Die Multiplex-Reaktion wurde zusammen mit den Sonden und Primem aus Fig. 4 durchgeführt. Für den Nachweis des eae-Gens wurden die Primer Nr. 68+73 und die Sonden Nr. 93+94 verwendet. Die Sonden Nr. 93+94 waren mit den Farbstoffen Fluorescein und Lightcycler Red705 gekoppelt. Die Detektion erfolgte bei einer Lichtwellenlänge von 710 nm.
Es sind zwei Kurvenscharen zu erkennen. Die Kurven mit Amplituden >5 zeigen ein positives Resultat für das eae-Gen. Hierbei handelt es sich um Stämme, die ein eae-Gen besitzen (s. Legende Fig.4, Tabelle S. 56). Die Kurven mit Amplituden <5 zeigen ein negatives Resutat (Wasserproben oder Stämme ohne eae-Gen (Proben 1, 11, 21, 5, 15, 25).

### Nachweis von VTEC-Stämmen durch PCR

Die vorliegende Erfindung illustriert den Nachweis von VTEC-Stämmen durch die Polymerasekettenreaktion. Bezogen auf das gesamte Genom unterscheiden sich VTEC-Stämme nur geringfügig von herkömmlichen *E. coli*-Stämmen. Aus diesem Grunde ist es nicht einfach die DNA- oder RNA-Sequenzen zu identifizieren, die die VTEC-Gruppe eindeutig abbilden. Da die VTEC auch in sich Unterschiede, z.B. in den Serotypen aufweisen, ist ein einzelnes Sequenzmerkmal nicht geeignet einen eindeutigen Nachweis zu liefern.

Die Erfindung beruht darauf, dass eine Kombination von mehreren genotypischen Merkmalen z.T. simultan und soweit notwendig z.T. konsekutiv für den Nachweis genutzt wird. Außerdem werden Primer und Sonden bereitgestellt, die die Vorteile der PCR für die Amplifikation und Detektion der VTEC-Stämme nutzen.

Der Nachweis der VTEC-Stämme kann in verschiedenen Schritten, umfassend Bakterienanreicherung, DNA-/RNA-Freisetzung und Isolierung, PCR und (eventuell simultan) Nachweis der Amplikons erfolgen.

Zur Anreicherung werden die Bakterien in 2 ml LB-Medium (10 g Bacto Trypton, 5 g Yeast Extrakt, 10 gNaCl in 1 l Wasser) ÜN bei 37°C geschüttelt. Die Bakterienkultur wurde dann bei 10000xg abzentrifugiert und in 100µl Wasser resuspendiert. Anschließend wurde 50µl 100 mM NaOH hinzugegeben. Die Zellen waren nach 5 min lysiert. Dann wurde die Lösung mit 100 µl 0,5 M Tris pH 8 neutralisiert. Abschliessend wurde die Suspension 10 min bei 10000xg zentrifugiert, um unlösliche Bestandteile zu entfernen. Von dieser Lösung wurde jeweils 1µl in die PCR-Reaktionen eingesetzt.

Die PCR-Reaktion wurde wie folgt angesetzt:
Probenvolumen - 1 µl
10xPCR-Puffer - 2,5 µl
10 mM dNTP - 0,25 µl
10 µM Vorwärtsprimer
   Kategorie A - 0,2 µl
10 µM Rückwärtsprimer
   Kategorie A - 0,2 µl
10 µM Vorwärtsprimer
   Kategorie B - 0,2 µl
10 µM Rückwärtsprimer
   Kategorie B - 0,2 µl
10 µM Vorwärtsprimer
   Kategorie C - 0,2 µl
10 µM Rückwärtsprimer
   Kategorie C - 0,2 µl
50 mM MgCl₂ - 0,75 µl
5U/µl Taq-Polymerase - 0,3 µl
Wasser - add. 25 µl

Das obige Reaktionsgemisch wurde in 200µl-Reaktionsgefässen fest verschlossen und gemäss nachfolgendem Protokoll in einem PCR-Gerät inkubiert:
95° C - 5 min 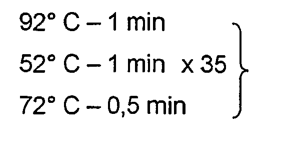
72° C - 5 min

In dem Reaktionsgemisch wurden jeweils ein Vorwärts- und ein Rückwärtsrprimer der Kategorie A, B, C (Tab. 1-9) eingesetzt. Beispielsweise wurden mit den Primern Nr. 1, 6, 18, 22, 26 und 30 Amplikons bei den in der nachfolgenden Tabelle aufgelisteten Stämmen erzeugt. Positive Ergebnisse lagen also bei diesen serologisch als VTEC klassifizierten Stämmen vor, da in jedem Fall durch PCR erzeugte Banden im Ethidiumbromid-gefärbten 1%-igen Agarosegel zu erkennen waren.

**Tab.: Nachweis von VTEC-Stämmen mit den Primem der Kategorie A-C**

| | **Stammnr. (Biotecon Diagnostics)** | **VTEC-Serotyp** | **Ergebnis positiv (+) /negativ(-)** |
|---|---|---|---|
| 1 | Bc 4734 | O26:H11 | + |
| 2 | Bc 4735 | O157:H- | + |
| 3 | Bc 4736 | | + |
| 4 | Bc 4737 | | + |
| 5 | Bc 4738 | O157:H7 | + |
| 6 | Bc 4945 | O26:H- | + |
| 7 | Bc 4946 | O157:H7 | + |
| 8 | Bc 4947 | O111:H- | + |
| 9 | Bc 4948 | O157:H | + |
| 10 | Bc 4949 | O5 | + |
| 11 | Bc 5643 | O2:H5 | + |
| 12 | Bc 5644 | O128 | + |
| 13 | Bc 5645 | O55:H- | + |
| 14 | Bc 5646 | O69:H- | + |
| 15 | Bc 5647 | O101:H9 | + |
| 16 | Bc 5648 | O103:H2 | + |
| 17 | Bc 5850 | O22:H8 | + |
| 18 | Bc 5851 | O55:H- | + |
| 19 | Bc 5852 | O48:H21 | + |
| 20 | Bc 5853 | O26:H11 | + |
| 21 | Bc 5854 | O157:H7 | + |
| 22 | Bc 5855 | O157:H- | + |
| 23 | Bc 5856 | O26:H- | + |
| 24 | Bc 5857 | O103:H2 | + |
| 25 | Bc 5858 | O26:H11 | + |
| 26 | Bc 7832 | | + |
| 27 | Bc 7833 | O Rauh- | + |
| | | form:H- | |
| 28 | Bc 7834 | ONT:H- | + |
| 29 | Bc 7835 | O103:H2 | + |
| 30 | Bc 7836 | O57:H- | + |
| 31 | Bc 7837 | ONT:H- | + |
| 32 | Bc 7838 | | + |
| 33 | Bc 7839 | O128:H2 | + |
| 34 | Bc 7840 | O157:H- | + |
| 35 | Bc 7841 | O23:H- | + |
| 36 | Bc 7842 | O157:H- | + |
| 37 | Bc 7843 | | + |
| 38 | Bc 7844 | O157:H- | + |
| 39 | Bc7845 | O103:H2 | + |
| 40 | Bc 7846 | O26:H11 | + |
| 41 | Bc7847 | O145:H- | + |
| 42 | Bc 7848 | O157:H- | + |
| 43 | Bc 7849 | O156:H47 | + |
| 44 | Bc 7850 | | + |
| 45 | Bc 7851 | O157:H- | + |
| 46 | Bc 7852 | O157:H- | + |
| 47 | Bc 7853 | O5:H- | + |
| 48 | Bc 7854 | O157:H7 | + |
| 49 | Bc 7855 | O157:H7 | + |
| 50 | Bc 7856 | O26:H- | + |
| 51 | Bc 7857 | | + |
| 52 | Bc 7858 | | + |
| 53 | Bc 7859 | ONT:H- | + |
| 54 | Bc 7860 | O129:H- | + |
| 55 | Bc 7861 | | + |
| 56 | Bc 7862 | O103:H2 | + |
| 57 | Bc 7863 | | + |
| 58 | Bc 7864 | O Rauh- | + |
| | | form:H- | |
| 59 | Bc 7865 | | + |
| 60 | Bc 7866 | O26:H- | + |
| 61 | Bc 7867 | O Rauh- | + |
| | | form: H- | |
| 62 | Bc 7868 | | + |
| 63 | Bc 7869 | ONT:H- | + |
| 64 | Bc 7870 | O113:H- | + |
| 65 | Bc 7871 | ONT:H- | + |
| 66 | Bc 7872 | ONT:H- | + |
| 67 | Bc7873 | | + |
| 68 | Bc 7874 | O Rauh- | + |
| | | form:H- | |
| 69 | Bc 7875 | O157:H- | + |
| 70 | Bc 7876 | O111:H- | + |
| 71 | Bc 7877 | O146:H21 | + |
| 72 | Bc 7878 | O145:H- | + |
| 73 | Bc 7879 | O22:H8 | + |
| 74 | Bc 7880 | O Rauh- | + |
| | | form:H- | |
| 75 | Bc 7881 | O145:H- | + |
| 76 | Bc 8275 | O157:H7 | + |
| 77 | Bc 8318 | O55:K-:H- | + |
| 78 | Bc 8325 | O157:H7 | + |
| 79 | Bc 8333 | | + |
| 80 | Bc 8332 | ONT | + |
| 81 | Bc 5580 | O157:H7 | + |
| 82 | Bc 5582 | O3: H | + |
| 83 | Bc 5579 | O157:H7 | + |

Außerdem konnten die Amplikons mit fluoreszensmarkierten Sondenpaaren aus den Kategorien A, B, C, also z.B. mit den Sonden SEQ ID Nr. 9,10, 34, 35, 95, 96, 97, 98 und 40+41 nachgewiesen werden.

### Nachweis von EHEC-Stämmen durch PCR

Enterohämorrhagische *E. coli* können als lebensmittelverderbende Keime schwere Durchfallerkrankungen hervorrufen. Sie sind verantwortlich für das HUS (hämolytisch-urämisches Syndrom), das eine blutige Diarrhöe und akutes Nierenversagen impliziert. Die Erkrankung kann tödlich enden.

Die EHEC können systematisch als eine Untergruppe der VTEC angesehen werden. Aus diesem Grunde kann der Nachweis in zwei Schritten erfolgen, in welchen zuerst die VTEC gemäss Beispiel 1 nachgewiesen werden und aus den positiven Befunden dann der EHEC-Nachweis erfolgt.

Im vorliegenden Beispiel werden Stämme der nachfolgenden Tabelle untersucht:

| Nr. | Biotecon Nr. | Serovar | VTEC +/- | EHEC+/- |
|---|---|---|---|---|
| 1 | BC 12503 | O157H- | + | + |
| 2 | BC 12507 | O157H- | + | + |
| 3 | BC 12408 | O84H21 | + | + |
| 4 | BC 12518 | O157H7 | + | + |
| 5 | BC 12530 | O156H- | + | + |
| 6 | BC 12538 | O157H7 | + | + |
| 7 | BC 12543 | O111H- | + | + |
| 8 | BC 12544 | O26H11 | + | + |
| 9 | BC 12545 | O103H2 | + | + |
| 10 | BC 12546 | O118H- | + | + |
| 11 | BC 12547 | O118H- | + | + |

Der Nachweis der EHEC-Stämme kann in verschiedenen Schritten, umfassend Bakterienanreicherung, DNA-/RNA-Freisetzung und Isolierung, PCR und (eventuell simultan) Nachweis der Amplikons erfolgen.

Zur Anreicherung werden die Bakterien in 2 ml LB-Medium (10 g Bacto Trypton, 5 g Yeast Extrakt, 10 g NaCl in 1 l Wasser) ÜN bei 37°C geschüttelt. Die Bakterienkultur wurde dann bei 10000xg abzentrifugiert und in 100µl Wasser resuspendiert. Anschließend wurde 50µl 100 mM NaOH hinzugegeben. Die Zellen waren nach 5 min lysiert. Dann wurde die Lösung mit 100 µl 0,5 M Tris pH 8 neutralisiert. Abschliessend wurde die Suspension 10 min bei 10000xg zentrifugiert, um unlösliche Bestandteile zu entfernen. Von dieser Lösung wurde jeweils 1µl in die PCR-Reaktionen eingesetzt.

Die PCR-Reaktion wurde wie folgt angesetzt:
Probenvolumen - 1 µl
1 0xPCR-Puffer - 2,5 µl
10 mM dNTP - 0,25 µl
10 µM Vorwärtsprimer
   Kategorie A - 0,2 µl
10 µM Rückwärtsprimer
   Kategorie A - 0,2 µl
10 µM Vorwärtsprimer
   Kategorie B - 0,2 µl
10 µM Rückwärtsprimer
   Kategorie B - 0,2 µl
10 µM Vorwärtsprimer
   Kategorie C - 0,2 µl
10 µM Rückwärtsprimer
   Kategorie C - 0,2 µl
50 mM MgCl₂- 0,75 µl
5U/µl Taq-Polymerase - 0,3 µl
Wasser - add. 25 µl

Das obige Reaktionsgemisch wurde in 200µl-Reaktionsgefässen fest verschlossen und gemäss nachfolgendem Protokoll in einem PCR-Gerät inkubiert:
95° C-5 min 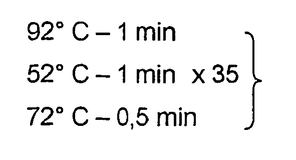
72° C - 5 min

In dem Reaktionsgemisch wurden jeweils ein Vorwärts- und ein Rückwärtsrprimer der Kategorie A, B, C (Tab. 1-9) eingesetzt. Beispielsweise wurden mit den Primern Nr. 1, 6, 18, 22, 26 und 30 Amplikons erzeugt. Positive Ergebnisse lagen also bei diesen serologisch als EHEC klassifizierten Stämmen vor, da in jedem Fall durch PCR erzeugte Banden im Ethidiumbromid-gefärbten 1%-igen Agarosegel zu erkennen waren.

Die DNA der positiven Befunde wurde erneut in einem zweiten Ansatz untersucht. In diesem wird eine PCR mit Vorwärts- und Rückwärtsprimer der Kategorien D und E eingesetzt. Es wird das nachfolgende Protokoll verwendet:
Probenvolumen - 1µl
10xPCR-Puffer - 2,5 µl
10 mM dNTP - 0,25 µl
10 µM Vorwärtsprimer
   Kategorie C - 0,2 µl
10 µM Rückwärtsprimer
   Kategorie C - 0,2 µl
10 µM Vorwärtsprimer
   Kategorie D - 0,2 µl
10 µM Rückwärtsprimer
   Kategorie D - 0,2 µl
50 mM MgCl₂ - 0,75 µl
5U/µl Taq-Polymerase - 0,3 µl
Wasser - add. 25 µl

Das obige Reaktionsgemisch wurde in 200µl-Reaktionsgefässen fest verschlossen und gemäss nachfolgendem Protokoll in einem PCR-Gerät inkubiert:
95° C - 5 min 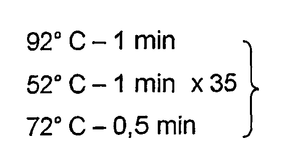
72° C - 5 min

Als Primer der Kategorie D kann z.B. die Kombination Primer Nr. 46, 54 und Nr. 68, 73 verwendet werden. Es ist auch möglich dieses Primerpaar in parallelen PCR-Reaktionen einzusetzen. Die Ergebnisse von zwei separaten PCR-Ansätzen sind nachfolgend dargestellt.

Da die Banden der Figuren 2 und 3 unterschiedliche Größen haben, können sie auch in einem Gel, aus einer einzigen PCR-Reaktion stammend, als Doppelbande nachgewiesen werden. Darüber hinaus können die Banden durch die zuvor beschriebene FRET-Technologie detektiert werden, indem Sondenpaare der Kategorien D und E verwendet werden. Beispielsweise können also die Sonden Nr. 60, 61 und 78,79 für diesen Zweck verwendet werden.

### Spezifität des EHEC-Nachweises

Wie zuvor beschrieben erfolgt der EHEC-Nachweis vorzugsweise in mindestens zwei Schritten, umfassend PCR-Reaktionen mit den Primer-Kategorien A-C und D-E. Dabei werden positive Resultate aus dem ersten Schritt in dem zweiten weiter untersucht. Falls der erste Schritt negativ ausfällt, kann dieses Ergebnis durch eine entsprechende Kontrolle, in welcher *E*. *coli* nachgewiesen wird, überprüft werden. Es ist darüber hinaus wichtig, dass die Primer der Kategorien A-C keine falsch-positiven Ergebnisse anzeigen. Aus diesem Grunde wurde ihre Spezifität intensiv untersucht. Nachfolgend sind die Ergebnisse dargestellt.

Zur Anreicherung werden die Bakterien in 2 ml LB-Medium (10 g Bacto Trypton, 5 g Yeast Extrakt, 10 gNaCl in 1 l Wasser) ÜN bei 37°C geschüttelt. Die Bakterienkultur wurde dann bei 10000xg abzentrifugiert und in 100µl Wasser resuspendiert. Anschließend wurde 50µl 100 mM NaOH hinzugegeben. Die Zellen waren nach 5 min lysiert. Dann wurde die Lösung mit 100 µl 0,5 M Tris pH 8 neutralisiert. Abschliessend wurde die Suspension 10 min bei 10000xg zentrifugiert, um unlösliche Bestandteile zu entfernen. Von dieser Lösung wurde jeweils 1µl in die PCR-Reaktionen eingesetzt.

Die PCR-Reaktion wurde wie folgt angesetzt:
Probenvolumen - 1 µl
10xPCR-Puffer - 2,5 µl
10 mM dNTP - 0,25 µl
10 µM Vorwärtsprimer
   Kategorie A - 0,2 µl
10 µM Rückwärtsprimer
   Kategorie A - 0,2 µl
10 µM Vorwärtsprimer
   Kategorie B - 0,2 µl
10 µM Rückwärtsprimer
   Kategorie B - 0,2 µl
10 µM Vorwärtsprimer
   Kategorie C - 0,2 µl
10 µM Rückwärtsprimer
   Kategorie C - 0,2 µl
50 mM MgCl₂ - 0,75 µl
5U/µl Taq-Polymerase - 0,3 µl
Wasser - add. 25 µl

Das obige Reaktionsgemisch wurde in 200µl-Reaktionsgefässen fest verschlossen und gemäss nachfolgendem Protokoll in einem PCR-Gerät inkubiert:
95° C - 5 min 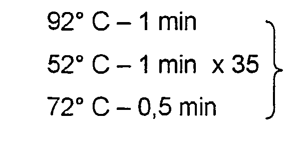
72° C - 5 min

In dem Reaktionsgemisch wurden jeweils ein Vorwärts- und ein Rückwärtsrprimer der Kategorie A, B, C (Tab. 1-9) eingesetzt. Beispielsweise wurden mit den Primern Nr. 1, 6, 18, 22, 26 und 30 keine Amplikons bei den in der nachfolgenden Tabelle aufgelisteten Stämmen erzeugt. Negative Ergebnisse lagen also bei diesen Stämmen vor, da in keinem Fall durch PCR erzeugte Banden der erwarteten Größe im Ethidiumbromid-gefärbten 1%-igen Agarosegel zu erkennen waren. Da die korrekten DNA-Fragmente nicht amplifiziert wurden, kann auch durch Sonden der Kategorien A-C kein falsch-positiver Befund entstehen. Auch dies wurde experimentell verifiziert.

**Tab.: Als Negativkontrollen getestete Bakterienstämme**

| Spezies | Stamm-Nr. | PCR-Nachweis |
|---|---|---|
| Aeromonas hydrophila | DSM30188 | - |
| Pseudomonas cepacia | BC 3134 | - |
| Pseudomonas paucimobilis | DSM1098 | - |
| Lactobacillus bifermentans | BC8463 | - |
| Flavobacterium johnsonii | DSM 2064 | - |
| Flavobacterium flavense | DSM 1076 | - |
| Flavobacterium resinovorum | DSM 7478 | - |
| Enterococcus casseliflavus | BC 7629 | - |
| Comamonas testosteroni | BC 4276 | - |
| Alcaligenes latus | DSM 1122 | - |
| Budvicia aquatica | BC 8923 | - |
| Achromobacter ruhlandii | BC 8908 | - |
| Achromobacter xylosa | BC 8913 | - |
| Sphingobacterium multivorans | BC 8924 | - |
| Ralstonia pickettii | BC 5368 | - |
| Sphingomonas paucimobilis | BC 5293 | - |
| Acinetobacter calcoaceticus | DSM 590 | - |
| Aeromonas hydrophila | DSM 6173 | - |
| Aeromonas enteropeloges | DSM 6394 | - |
| Moraxella catarrhalis | DSM 9143 | - |
| Pasteurella pneumotropica | DSM 2891 | - |
| Pseudomonas beijerinkii | DSM 7218 | - |
| Stenotrophomonas putrefaciens | BC 5337 | - |
| Xanthomonas maltophila | BC 4273 | - |
| Brochotrix thermosphacta | DSM 20171 | - |
| Brochotrix thermophilus | DSM 20594 | - |
| Brochotrix campestris | DSM 4712 | - |
| Staphylococcus haemolyticus | BC 2747 | - |
| Staphylococcus chromogenes | BC 5468 | - |
| Staphylococcus gallinosum | BC5472 | - |
| Staphylococcus lentus | BC 5462 | - |
| Staphylococcus intermedius | DSM 20036 | - |
| Staphylococcus saprophyticus | DSM 20038 | - |
| Staphylococcus hominis | BC 5466 | - |
| Staphylococcus equorum | BC 9447 | - |
| Staphylococcus sciuri | BC 5461 | - |
| Staphylococcus hyicus | BC 5469 | - |
| Aeromonas caviae | DSM 7326 | - |
| Pantoea stewartii | DSM 30176 | - |
| Xhenorhabdus poinarii | DSM 4768 | - |
| Klebsiella ornitholytica | DSM 7464 | - |
| Vibrio vulnificus | DSM 10147 | - |
| Moellerella wisconsis | DSM 5079 | - |
| Yersinia pseudotuberculosis | BC 8723 | - |
| Vibrio mimicus | DSM 33653 | - |
| Aeromonas sobriae | ATCC 43979 | - |
| Pasteurella aerogenes | DSM 10153 | - |
| Listonella anguillarum | DSM11323 | - |

### Verwendung einer E. coli-Positivkontrolle

Wie zuvor beschrieben werden EHEC-Stämme gemäss der vorliegenden Erfindung in zwei Schritten unter Verwendung der Primer A-C und D-E nachgewiesen. Sobald PCR-Reaktionen des ersten Schritts einen positiven Befund anzeigen, werden die Proben nach Schritt zwei weiter untersucht. Wenn hingegen Schritt eins negativ ausfällt, dann liegt kein VTEC, und damit ebenfalls kein EHEC-Stamm vor. Es muss jedoch sichergestellt werden, dass experimentell bedingte Fehler ausgeschlossen werden können. Eine Möglichkeit besteht darin, *E*. *coli* nachzuweisen, da dieser Keim in fast allen für EHEC relevanten Nahrungsmitteln vorkommt. Durch Dotierung eines Lebensmittels mit einem *E. coli*-Stamm ist die Möglichkeit gegeben, diesen ungefährlichen Kontrollkeim routinemässig zu verwenden. Außerdem ist ein Nachweis von *E*. *coli* aus hygienischen Gesichtspunkten häufig erwünscht.

Aus Reinkulturen der in der nachfolgenden Tabelle aufgeführten Bakterien wurde in an sich bekannten Standardverfahren genomische DNA isoliert. Je ca. 1 bis 10 ng dieser Präparationen wurde dann in Gegenwart von je 0,4 µM eines äquimolaren Oligonukleotidgemisches Nr.84-87 und 0,4 µM Oligonukleotid Nr. 88, 2 mM MgCl₂, 200 µM dNTPs (Roche Diagnostics, statt dTTP' wurde dUTP verwendet), und 0,03 U/µl Taq Polymerase (Life Technologies) in einfach konzentrierten Reaktionspuffer (Life Technologies) in die PCR eingesetzt. Die PCR wurde in einem Perkin Elmer 9600 Thermocycler mit nachfolgend aufgeführten Thermoprofil durchgeführt:

| | | |
|---|---|---|
| initiale Denaturierung | 95 °C | 5 min |
| Amplifikation (35 Zyklen) | 95 °C | 20 sec |
| | 63 °C | 45 sec |
| finale Synthese | 72 °C | 5 min |

Nach Beendigung der PCR-Reaktion wurden die Amplifikationsprodukte mittels Agarose-Gelelektrophorese aufgetrennt und durch Anfärbung mit Ethidiumbromid sichtbar gemacht. Die erwarteten Produkte von 351 Basenpaare Länge wurden nur in den Fällen beobachtet, in denen DNA von Stämmen der Spezies *E*. *coli* bzw. der Gattung *Shigella* anwesend war. Die in den Gelen aufgetrennte DNA wurde in an sich bekannten Standardverfahren auf Nylon Filter transferiert und zur Überprüfung der Spezifität mit dem am 5`-Ende Biotin-markierten Oligonukleotiden Nr.91 und Nr.92 hybridisiert. Die Hybridisierung erfolgte in 5 x SSC, 2 % Blocking Reagenz, 0,1 % Lauroylsarcosin, 0,02 % SDS und 5 pmol/ml Sonde für 4 h bei 52°C. Gewaschen wurde in 2 x SSC, 0,1 % SDS für 2 x 10 min bei 52 °C. Die Detektion erfolgte in an sich bekannten Standardverfahren mittels Alkalischer Phosphatase-Konjugate (ExtrAvidin, Sigma) in Anwesenheit von 5-Bromo-4chloro-3-indolylphosphat und 4-Nitro-Blue Tetrazoliumchlorid (Boehringer Mannheim). Auf den Filtern wurde nur in den Fällen eine Bande beobachtet, in denen zuvor eine Bande von 351 Basenpaaren auf dem Agarosegel sichtbar war. Somit wurde mittels PCR und Hybridisierung die Anwesenheit sämtlicher 645 getesteten *E*. *coli* und 32 *Shigella*-Stämme nachgewiesen (siehe nachfolgende Tabelle). Hingegen wurde keiner der getesteten nicht zu dieser Spezies gehörenden Bakterienstämme mit diesem System erfasst.

**Tabelle: Liste der getesteten Bakterien der E. coli/Shigella-Gruppe**

| **Spezies** | **Stamm-Nr.** | **Serotyp** | **Pathotyp** | **PCR-Nachweis** | **Hybridisierung mit Sonden** |
|---|---|---|---|---|---|
| *E. coli* | NCTC 12757 | n.b. | | + | + |
| *E. coli* | NCTC 12779 | n.b. | | + | + |
| *E. coli* | NCTC 12790 | n.b. | | + | + |
| *E. coli* | NCTC 12796 | n.b. | | + | + |
| *E. coli* | NCTC 12811 | n.b. | | + | + |
| *E. coli* | ATCC 11229 | n.b. | | + | + |
| *E. coli* | ATCC 25922 | n.b. | | + | + |
| *E. coli* | ATCC 8739 | n.b. | | + | + |
| *E. coli* | DSM 30083 | O1:K1:H7 | | + | + |
| *E. coli* | BC 5849 | O111:H2 | | + | + |
| *E. coli* | BC 8265 | O104 | | + | + |
| *E. coli* | BC 8267 | O55 | | + | + |
| *E. coli* | BC 8268 | O6:H16 | | + | + |
| *E. coli* | BC 8270 | O55:K(59):H- | | + | + |
| *E. coli* | BC 8271 | O55 | | + | + |
| *E*. *coli* | BC 8272 | O55:K-:H- | | + | + |
| *E. coli* | BC 8273 | O55 | | + | + |
| *E. coli* | BC 8276 | O128:K-H- | | + | + |
| *E. coli* | BC 8277 | O128:K68:H2 | | + | + |
| *E. coli* | BC 8278 | O126 | | + | + |
| *E. coli* | BC 8279 | O126 | | + | + |
| *E. coli* | BC 8312 | ONT:H- | | + | + |
| *E. coli* | BC 8317 | O158:K-:H23 | | + | + |
| *E. coli* | BC 8319 | O128:H21 | | + | + |
| *E. coli* | BC 8320 | O55:H- | | + | + |
| *E. coli* | BC 8321 | O55 | | + | + |
| *E. coli* | BC 8322 | O55 | | + | + |
| *E. coli* | RC 8326 | O104 | | + | + |
| *E. coli* | BC 8327 | O37 | | + | + |
| *E. coli* | BC 8331 | O24 | | + | + |
| *E. coli* | BC 8335 | O119:H27 | | + | + |
| *E. coli* | BC 8338 | O10:H4 | | + | + |
| *E. coli* | BC 8341 | O110:H17 | | + | + |
| *E. coli* | BC 8344 | O103 | | + | + |
| *E. coli* | BC 8345 | O103 | | + | + |
| *E. coli* | BC 8346 | O44 | | + | + |
| *E. coli* | RC 8347 | O44 | | + | + |
| *E*. *coli* | BC 8348 | O44 | | + | + |
| *E*. *coli* | BC 8863 | n.b. | | + | + |
| *E*. *coli* | BC 8864 | n.b. | | + | + |
| *E*. *coli* | BC 4734 | O26:H11 | VTEC | + | + |
| *E. coli* | BC 4735 | O157:H- | VTEC | + | + |
| *E coli* | BC 4736 | n.b. | VTEC | + | + |
| *E*. *coli* | BC 4737 | n.b. | VTEC | + | + |
| *E*. *coli* | BC 4738 | O157:H7 | VTEC | + | + |
| *E. coli* | BC 4945 | O26:H- | VTEC | + | + |
| *E*. *coli* | BC 4946 | O157:H7 | VTEC | + | + |
| *E*. *coli* | BC 4947 | O111:H- | VTEC | + | + |
| *E*. *coli* | BC 4948 | O157:H | VTEC | + | + |
| *E*. *coli* | BC 4949 | O5 | VTEC | + | + |
| *E*. *coli* | BC 5579 | O157:H7 | VTEC | + | + |
| *E*. *coli* | BC 5580 | O157:H7 | VTEC | + | + |
| *E*. *coli* | BC 5582 | O3:H | VTEC | + | + |
| *E*. *coli* | BC 5643 | O2:H5 | VTEC | + | + |
| *E*. *coli* | BC 5644 | O128 | VTEC | + | + |
| *E*. *coli* | BC 5645 | O55:H- | VTEC | + | + |
| *E*. *coli* | BC 5646 | O69:H- | VTEC | + | + |
| *E*. *coli* | BC 5647 | O101:H9 | VTEC | + | + |
| *E*. *coli* | BC 5648 | O103:H2 | VTEC | + | + |
| *E*. *coli* | BC 5850 | O22:H8 | VTEC | + | + |
| *E*. *coli* | BC 5851 | O55:H- | VTEC | + | + |
| *E*. *coli* | BC 5852 | O48:H21 | VTEC | + | + |
| *E*. *coli* | BC 5853 | O26:H11 | VTEC | + | + |
| *E*. *coli* | BC 5854 | O157:H7 | VTEC | + | + |
| *E*. *coli* | BC 5855 | O157:H- | VTEC | + | + |
| *E*. *coli* | BC 5856 | O96:H- | VTFC | + | + |
| *E. coli* | BC 5857 | O103:H2 | VTEC | + | + |
| *E*. *coli* | BC 5858 | O26:H11 | VTEC | + | + |
| *E*. *coli* | BC 7832 | n.b. | VTEC | + | + |
| *E*. *coli* | BC 7833 | O Rauhform:H- | VTFC | + | + |
| *E*. *coli* | BC 7834 | ONT:H- | VTEC | + | + |
| *E*. *coli* | BC 7835 | O103:H2 | VTEC | + | + |
| *E*. *coli* | BC 7836 | O57:H- | VTEC | + | + |
| *E*. *coli* | BC 7837 | ONT:H- | VTFC | + | + |
| *E*. *coli* | BC 7838 | n.b. | VTEC | + | + |
| *E*. *coli* | BC 7839 | O128:H2 | VTEC | + | + |
| *E*. *coli* | BC 7840 | O157:H- | VTEC | + | + |
| *E. coli* | BC 7841 | O23:H- | VTEC | + | + |
| *E. coli* | BC 7842 | O157:H- | VTEC | + | + |
| *E. coli* | BC 7843 | n.b. | VTEC | + | + |
| *E. coli* | BC 7844 | O157:H- | VTEC | + | + |
| *E. coli* | BC 7845 | O103:H2 | VTEC | + | + |
| *E. coli* | BC 7846 | O26:H11 | VTEC | + | + |
| *E. coli* | BC 7847 | O145:H- | VTEC | + | + |
| *E. coli* | BC 7848 | O157:H- | VTFC | + | + |
| *E. coli* | BC 7849 | O156:H47 | VTEC | + | + |
| *E. coli* | BC 7850 | n.b. | VTEC | + | + |
| *E. coli* | BC 7851 | O157:H- | VTEC | + | + |
| *E. coli* | BC 7852 | O157:H- | VTEC | + | + |
| *E. coli* | BC 7853 | O5:H- | VTEC | + | + |
| *E. coli* | BC 7854 | O157:H7 | VTEC | + | + |
| *E. coli* | BC 7855 | O157:H7 | VTEC | + | + |
| *E. coli* | BC 7856 | O26:H- | VTEC | + | + |
| *E. coli* | BC 7857 | n.b. | VTEC | + | + |
| *E. coli* | BC 7858 | n.b. | VTEC | + | + |
| *E. coli* | BC 7859 | ONT:H- | VTEC | + | + |
| *E. coli* | BC 7860 | O129:H- | VTEC | + | + |
| *E. coli* | BC 7861 | n.b. | VTEC | + | + |
| *E. coli* | BC 7862 | O103:H2 | VTEC | + | + |
| *E. coli* | BC 7863 | n.b. | VTEC | + | + |
| *E. coli* | BC 7864 | O Rauhform:H- | VTEC | + | + |
| *E. coli* | BC 7865 | n.b. | VTEC | + | + |
| *E. coli* | BC 7866 | O26:H- | VTEC | + | + |
| *E. coli* | BC 7867 | O Rauhform:H- | VTEC | + | + |
| *E. coli* | BC 7868 | n.b. | VTEC | + | + |
| *E. coli* | BC 7869 | ONT:H- | VTEC | + | + |
| *E. coli* | BC 7870 | O113:H- | VTEC | + | + |
| *E. coli* | BC 7871 | ONT:H- | VTEC | + | + |
| *E. coli* | BC 7872 | ONT:H- | VTEC | + | + |
| *E. coli* | BC 7873 | n.b. | VTEC | + | + |
| *E. coli* | BC 7874 | O Rauhform:H- | VTEC | + | + |
| *E. coli* | BC 7875 | O157:H- | VTEC | + | + |
| *E. coli* | BC 7876 | O111:H- | VTEC | + | + |
| *E. coli* | BC 7877 | O146:H21 | VTEC | + | + |
| *E. coli* | BC 7878 | O145:H- | VTEC | + | + |
| *E. coli* | BC 7879 | O22:H8 | VTEC | + | + |
| *E. coli* | BC 7880 | O Rauhform:H- | VTEC | + | + |
| *E. coli* | BC 7881 | O145:H- | VTEC | + | + |
| *E. coli* | BC 8275 | O157:H7 | VTEC | + | + |
| *E. coli* | BC 8318 | O55:K-:H- | VTEC | + | + |
| *E. coli* | BC 8325 | O157:H7 | VTEC | + | + |
| *E. coli* | BC 8332 | ONT | VTEC | + | + |
| *E. coli* | BC 8333 | n.b. | VTEC | + | + |
| *E. coli* | BC 8246 | O152:K-:H- | EIEC | + | + |
| *E. coli* | BC 8247 | O124:K(72):H3 | EIEC | + | + |
| *E. coli* | BC 8248 | O124 | EIEC | + | + |
| *E. coli* | BC 8249 | O112 | EIEC | + | + |
| *E. coli* | BC 8250 | O136:K(78):H- | EIEC | + | + |
| *E. coli* | BC 8251 | O124:H- | EIEC | + | + |
| *E. coli* | BC 8252 | O144:K-:H- | EIEC | + | + |
| *E. coli* | BC 8253 | O143:K:H- | EIEC | + | + |
| *E. coli* | BC 8254 | O143 | EIEC | + | + |
| *E. coli* | BC 8255 | O112 | EIEC | + | + |
| *E. coli* | BC 8256 | O28a,e | EIEC | + | + |
| *E. coli* | BC 8257 | O124:H- | EIEC | + | + |
| *E. coli* | BC 8258 | O143 | EIEC | + | + |
| *E. coli* | BC 8259 | O167:K-:H5 | EIEC | + | + |
| *E. coli* | BC 8260 | O128a.c.:H35 | EIEC | + | + |
| *E. coli* | BC 8261 | O164 | EIEC | + | + |
| *E. coli* | BC 8262 | O164:K-:H- | EIEC | + | + |
| *E. coli* | BC 8263 | O164 | EIEC | + | + |
| *E. coli* | BC 8264 | O124 | FIFC | + | + |
| *E. coli* | BC 7567 | O86 | EPEC | + | + |
| *E. coli* | BC 7568 | O128 | EPEC | + | + |
| *E. coli* | BC 7571 | O114 | EPEC | + | + |
| *E. coli* | BC 7572 | O119 | EPEC | + | + |
| *E. coli* | BC 7573 | O125 | EPEC | + | + |
| *E. coli* | BC 7574 | O124 | EPEC | + | + |
| *E. coli* | BC 7576 | O127a | EPEC | + | + |
| *E. coli* | BC 7577 | O126 | EPEC | + | + |
| *E. coli* | BC 7578 | O142 | EPEC | + | + |
| *E. coli* | BC 7579 | O26 | EPEC | + | + |
| *E. coli* | BC 7580 | OK26 | EPEC | + | + |
| *E. coli* | BC 7581 | O142 | EPEC | + | + |
| *E. coli* | BC 7582 | O55 | EPEC | + | + |
| *E. coli* | BC 7583 | O158 | EPEC | + | + |
| *E. coli* | BC 7584 | O- | EPEC | + | + |
| *E. coli* | BC 7585 | O- | EPEC | + | + |
| *E. coli* | BC 7586 | O- | EPEC | + | + |
| *E. coli* | BC 8330 | n.b. | EPEC | + | + |
| *E. coli* | BC 8550 | O26 | EPEC | + | + |
| *E*. *coli* | BC 8551 | O55 | EPEC | + | + |
| *E*. *coli* | BC 8552 | O158 | EPEC | + | + |
| *E*. *coli* | BC 8553 | O26 | EPEC | + | + |
| *E*. *coli* | BC 8554 | O158 | EPEC | + | + |
| *E. coli* | BC 8555 | O86 | EPEC | + | + |
| *E*. *coli* | BC 8556 | O128 | EPEC | + | + |
| *E*. *coli* | BC 8557 | OK26 | EPEC | + | + |
| *E. coli* | BC 8558 | O55 | EPEC | + | + |
| *E*. *coli* | BC 8560 | O158 | EPEC | + | + |
| *E*. *coli* | BC 8561 | O158 | EPEC | + | + |
| *E. coli* | BC 8562 | O114 | EPEC | + | + |
| *E. coli* | BC 8563 | O86 | EPEC | + | + |
| *E. coli* | BC 8564 | O128 | EPEC | + | + |
| *E. coli* | BC 8565 | O158 | EPEC | + | + |
| *E. coli* | BC 8566 | O158 | EPEC | + | + |
| *E. coli* | BC 8567 | O158 | EPEC | + | + |
| *E. coli* | BC 8568 | O111 | EPEC | + | + |
| *E. coli* | BC 8569 | O128 | EPEC | + | + |
| *E. coli* | BC 8570 | O114 | EPEC | + | + |
| *E. coli* | BC 8571 | O128 | EPEC | + | + |
| *E*. *coli* | BC 8572 | O128 | EPEC | + | + |
| *E*. *coli* | BC 8573 | O158 | EPEC | + | + |
| *E. coli* | BC 8574 | O158 | EPEC | + | + |
| *E. coli* | BC 8575 | O158 | EPEC | + | + |
| *E. coli* | BC 8576 | O158 | EPEC | + | + |
| *E*. *coli* | BC 8577 | O158 | EPEC | + | + |
| *E*. *coli* | BC 8578 | O158 | EPEC | + | + |
| *E*. *coli* | BC 8581 | O158 | EPEC | + | + |
| *E*. *coli* | BC 8583 | O128 | EPEC | + | + |
| *E. coli* | BC 8584 | O158 | EPEC | + | + |
| *E. coli* | BC 8585 | O128 | EPEC | + | + |
| *E. coli* | BC 8586 | O158 | EPEC | + | + |
| *E. coli* | BC 8588 | O26 | EPEC | + | + |
| *E. coli* | BC 8589 | O86 | EPEC | + | + |
| *E. coli* | BC 8590 | O127 | EPEC | + | + |
| *E. coli* | BC 8591 | O128 | EPEC | + | + |
| *E*. *coli* | BC 8592 | O114 | EPEC | + | + |
| *E. coli* | BC 8593 | O114 | FPFC | + | + |
| *E. coli* | BC 8594 | O114 | EPEC | + | + |
| *E. coli* | BC 8595 | O125 | EPEC | + | + |
| *E. coli* | BC 8596 | O158 | EPEC | + | + |
| *E. coli* | BC 8597 | O26 | EPEC | + | + |
| *E. coli* | BC 8598 | O26 | EPEC | + | + |
| *E. coli* | BC 8599 | O158 | EPEC | + | + |
| *E. coli* | BC 8605 | O158 | EPEC | + | + |
| *E. coli* | BC 8606 | O158 | EPEC | + | + |
| *E. coli* | BC 8607 | O158 | EPEC | + | + |
| *E. coli* | BC 8608 | O128 | EPEC | + | + |
| *E. coli* | BC 8609 | O55 | EPEC | + | + |
| *E*. *coli* | BC 8610 | O114 | EPEC | (+) | + |
| *E*. *coli* | BC 8615 | O158 | EPEC | + | + |
| *E. coli* | BC 8616 | O128 | EPEC | + | + |
| *E. coli* | BC 8617 | O26 | EPEC | + | + |
| *E*. *coli* | BC 8618 | O86 | EPEC | + | + |
| *E*. *coli* | BC 8619 | n.b. | EPEC | + | + |
| *E*. *coli* | BC 8620 | n.b. | EPEC | + | + |
| *E*. *coli* | BC 8621 | n.b. | EPEC | + | + |
| *E. coli* | BC 8622 | n.b. | EPEC | + | + |
| *E*. *coli* | BC 8623 | n.b. | EPEC | + | + |
| *E*. *coli* | BC 8624 | O158 | EPEC | + | + |
| *E*. *coli* | BC 8625 | O158 | EPEC | + | + |
| *E. coli* | BC 5581 | O78:H11 | ETEC | + | + |
| *E*. *coli* | BC 5583 | O2:K1 | ETEC | + | + |
| *E*. *coli* | BC 8221 | O118 | ETEC | + | + |
| *E*. *coli* | BC 8222 | O148:H- | ETEC | + | + |
| *E. coli* | BC 8223 | O111 | ETEC | + | + |
| *E. coli* | BC 8224 | O110:H- | ETEC | + | + |
| *E. coli* | BC 8225 | O148 | ETEC | + | + |
| *E*. *coli* | BC 8226 | O118 | ETEC | + | + |
| *E. coli* | BC 8227 | O25:H42 | ETEC | + | + |
| *E*. *coli* | BC 8229 | O6 | ETEC | + | + |
| *E*. *coli* | BC 8231 | O153:H45 | ETEC | + | + |
| *E. coli* | BC 8232 | O9 | ETEC | + | + |
| *E. coli* | BC 8233 | O148 | ETEC | + | + |
| *E*. *coli* | BC 8234 | O128 | ETEC | + | + |
| *E*. *coli* | BC 8235 | O118 | ETEC | + | + |
| *E*. *coli* | BC 8237 | O111 | ETEC | + | + |
| *E*. *coli* | BC 8238 | O110:H17 | ETEC | + | + |
| *E. coli* | BC 8240 | O148 | ETEC | + | + |
| *E. coli* | BC 8241 | O6H16 | ETEC | + | + |
| *E*. *coli* | BC 8243 | O153 | ETEC | + | + |
| *E. coli* | BC 8244 | O15:H- | ETEC | + | + |
| *E. coli* | BC 8245 | O20 | ETEC | + | + |
| *E*. *coli* | BC 8269 | O125a,c:H- | ETEC | + | + |
| *E. coli* | BC 8313 | O6:H6 | ETEC | + | + |
| *E*. *coli* | BC 8315 | O153:H- | ETEC | + | + |
| *E*. *coli* | BC 8329 | n.b. | ETEC | + | + |
| *E. coli* | BC 8334 | O118:H12 | ETEC | + | + |
| *E. coli* | BC 8339 | n.b. | ETEC | + | + |
| | | | | | |
| *E. coli* Klinische Isolate | | | | 359 (359) | 359 (359) |
| *E. coli* Lebensmittelisolate | | | | 12 (12) | 12 (12) |
| *E. coli* Umweltisolate | | | | 23 (23) | 23 (23) |
| | | | | | |
| *Shigella boydii* | DSM 7532 | 2 | | + | + |
| *Sh. boydii* | BC 7545 | 1 | | + | + |
| *Sh. boydii* | BC 7546 | 2 | | + | + |
| *Sh. boydii* | BC 7547 | 3 | | + | + |
| *Sh. boydii* | BC 7548 | 4 | | + | + |
| *Sh. boydii* | BC 7549 | 5 | | + | + |
| *Sh. boydii* | BC 7550 | 6 | | + | + |
| *Sh. boydii* | BC 7551 | 7 | | + | + |
| *Sh. boydii* | BC 7552 | 8 | | + | + |
| *Sh. dysenteriae* | NCTC 4837 | 1 | | + | + |
| *Sh. dysenteriae* | BC 7566 | 1 | | + | + |
| *Sh. dysenteriae* | BC 7553 | 2 | | + | + |
| *Sh. dysenteriae* | BC 7554 | 3 | | + | + |
| *Sh. dysenteriae* | BC 7555 | 5 | | + | + |
| *Sh. dysenteriae* | BC 7556 | 7 | | + | + |
| *Sh. dysenteriae* | BC 7557 | 8 | | + | + |
| *Sh. dysenteriae* | BC 7559 | 10 | | + | + |
| *Sh. flexneri* | DSM 4782 | 2a | | + | + |
| *Sh. flexneri* | BC 5935 | 1a | | + | + |
| *Sh. flexneri* | BC 5936 | 2a | | + | + |
| *Sh. flexneri* | BC 5937 | 6 | | + | + |
| *Sh. flexneri* | BC 7560 | 1b | | + | + |
| *Sh. flexneri* | BC 7561 | 2a | | + | + |
| *Sh. flexneri* | BC 7562 | 3b | | + | + |
| *Sh. flexneri* | BC 7563 | 4 | | + | + |
| *Sh. flexneri* | BC 7564 | 5 | | + | + |
| *Sh. flexneri* | BC 7565 | 6 | | + | + |
| *Shiagella sonnei* | BC 1201 | | | + | + |
| *Shigella sonnei* | BC 4302 | | | + | + |
| *Shigella sonnei* | BC 4301 | | | + | + |
| *Shigella sonnei* | BC 7889 | | | + | + |
| *Shigella sp.* | BC 4303 | | | + | + |

| | | | | | |
|---|---|---|---|---|---|
| ATCC: American Type Culture Collection (Manassas, USA) BC: Stammsammlung BioteCon GmbH DSM: Deutsche Sammlung von Mikroorganismen (Braunschweig, Deutschland) NCTC: National Collection of Type Cultures (London, Großbritannien) + = positive Reaktion - = negative Reaktion (+) = schwache positive Reaktion n.b. = nicht bestimmt | | | | | |

**Tabelle: Liste der getesteten Bakterien außerhalb der E. coli/Shigella-Gruppe**

| **Spezies** | **Stamm-Nr.** | **PCR-Nachweis** | **Hybridisierung mit Sonden** |
|---|---|---|---|
| *Buttiauxella agrestis* | DSM 4586 | - | - |
| *Cedecea davisae* | DSM 4568 | - | - |
| *Citrobacter amalonaticus* | DSM 4593 | - | - |
| *Citrobacter freundii* | DSM 30040 | - | - |
| *Citrobacter freundii* | BC 6044 | - | - |
| *Citrobacter koseri* | DSM 4570 | - | - |
| *Citrobacter koseri* | DSM 4595 | - | - |
| *Citrobacter koseri* | BC 4962 | - | - |
| *Edwartsiella tarda* | DSM 30052 | - | - |
| *Enterobacter aerogenes* | DSM 30053 | - | - |
| *Enterobacter aerogenes* | BC 5895 | - | - |
| *Enterobacter amnigenus* | DSM 4486 | - | - |
| *Enterobacter amnigenus* | BC 7437 | - | - |
| *Enterobacter amnigenus* | BC 8794 | - | - |
| *Enterobacter cloacae* | DSM 30054 | - | - |
| *Enterobacter cloacae* | BC 2467 | - | - |
| *Enterobacter cloacae* | BC 8725 | - | - |
| *Enterobacter gergoviae* | BC 511 | - | - |
| *Enterobacter gergoviae* | BC 674 | - | - |
| *Enterobacter intermedius* | DSM 4581 | - | - |
| *Enterobacter sakazakii* | DSM 4485 | - | - |
| *Erwinia carotovora subsp. carotovora* | DSM 30168 | - | - |
| *Escherichia blattae* | NCTC 12127 | - | - |
| *Escherichia hermannii* | DSM 4560 | - | - |
| *Escherichia hermannii* | BC 8467 | - | - |
| *Escherichia fergusonii* | NCTC 12128 | (+) | - |
| *Escherichia vulneris* | DSM 4564 | - | - |
| *Escherichia vulneris* | BC 8793 | - | - |
| *Hafnia alvei* | BC 2154 | - | - |
| *Klebsiella oxytoca* | DSM 5175 | - | - |
| *Klebsiella oxytoca* | BC 2468 | - | - |
| *Klebsiella planticola* | DSM 4617 | - | - |
| *Klebsiella pneumoniae* | BC 5365 | - | - |
| *Klebsiella pneumoniae subsp. pneumoniae* | ATCC 13883 | - | - |
| *Klebsiella pneumoniae subsp. pneumoniae* | DSM 30102 | - | - |
| *Klebsiella terrigena* | DSM 2687 | - | - |
| *Kluyvera ascorbata* | DSM 4611 | - | - |
| *Kluyvera sp.* | BC 7440 | - | - |
| *Morganella morganii subsp. morganii* | DSM 30164 | - | - |
| *Pantoea agglomerans* | DSM 3493 | - | - |
| *Pantoea agglomerans* | BC 6043 | - | - |
| *Pantoea agglomerans* | BC 8600 | - | - |
| *Pantoea spp.* | BC 8669 | - | - |
| *Pantoea spp.* | BC 8726 | - | - |
| *Proteus mirabilis* | DSM 788 | - | - |
| *Proteus rettgeri* | DSM 1131 | - | - |
| *Providencia stuartii* | DSM 4539 | - | - |
| *Rahnella aquatitis* | DSM 4594 | - | - |
| *Salmonella bongori* V | BC 5695 | - | - |
| *Salmonella bongori V* | BC 7952 | - | - |
| *Salmonella enterica I* | BC 7751 | - | - |
| *Salmonella enterica II* | BC 5677 | - | - |
| *Salmonella enterica IIIa* | BC 5241 | - | - |
| *Salmonella enterica IIIa* | BC 5249 | - | - |
| *Salmonella enterica IIIb* | BC 7937 | - | - |
| *Salmonella enterica IIIb* | BC 7942 | - | - |
| *Salmonella enterica IV* | BC 7759 | - | - |
| *Salmonella enterica VI* | BC 7762 | - | - |
| *Serratia marcescens* | BC 677 | - | - |
| *Serratia marcescens* | DSM1636 | - | - |
| *Serratia odorifera* | BC 678 | - | - |
| *Serratia spp.* | BC 1139 | - | - |
| *Yersinia enterocolytica* | DSM 4780 | - | - |
| *Yersinia pseudotuberculosis* | DSM 8992 | - | - |
| *Yokenella regensburgei* | DSM 5079 | - | - |
| *Acinetobacter sp.* | DSM590 | - | - |
| *Aeromonas hydrophila subsp. hydrophila* | DSM 6173 | - | - |
| *Bacillus cereus* | NCFB 827 | - | - |
| *Bacillus stearothermophilus* | DSM 1550 | - | - |
| *Bacillus subtilis* | DSM 1970 | - | - |
| *Carnobacterium mobile* | DSM 4848 | - | - |
| *Clostridium acetobutylicum* | DSM 1731 | - | - |
| *Clostridium propionicum* | DSM1682 | - | - |
| *Clostridium saccharolyticum* | DSM 2544 | - | - |
| *Comamonas testosteroni* | DSM1622 | - | - |
| *Enterococcus faecalis* | DSM 6134 | - | - |
| *Flavobacterum sp.* | ATCC 27551 | - | - |
| *Haemophilus influenzae* | DSM 4690 | - | - |
| *Lactococcus lactis subsp. hordniea* | DSM 20450 | - | - |
| *Lactococcus raffinolactis* | DSM 20443 | - | - |
| *Moraxella catarrhalis* | DSM 9143 | - | - |
| *Pasteurella pneumotropica* | BC 2891 | - | - |
| *Pediococcus inopinatus* | DSM 20285 | - | - |
| *Pseudomonas aeruginosa* | DSM 50071 | - | - |
| *Pseudomonas cepacia* | BC 3134 | - | - |
| *Pseudomonas fluorescens* | DSM 6290 | - | - |
| *Sphingomonas paucimobilis* | BC 8795 | - | - |
| *Sphingomonas sp.* | DSM 6014 | - | - |
| *Staphylococcus aureus subsp. aureus* | DSM 20491 | - | - |
| *Stenotrophomonas maltophila* | BC 8724 | - | - |
| *Streptococcus thermophilus* | BC 2148 | - | - |
| *Vibrio alginolyticus* | DSM 2171 | - | - |
| *Vibrio fischeri* | DSM 507 | - | - |
| *Vibrio harveyi* | DSM 6904 | - | - |
| *Vibrio parahaemolyticus* | DSM 2172 | - | - |

### Differenzierung von Sit-Genen

Ein charakteristisches Kennzeichen der VTEC ist das Vorhandensein eines der beiden Gene Sltl (Shiga like toxin) oder Sltll oder von beiden Genen. Diese Gene werden auch als vtx1 und vtx2 bezeichnet. Zur Feintypisierung von VTEC und EHEC-Stämmen kann bezüglich des Vorhandenseins dieser Gene oder von Varianten dieser Gene weiter differenziert werden. Auf diese Weise lassen sich wichtige Informationen zur Verbreitung dieser pathogenen *E. coli*-Stämme wie auch zur Evolution gewinnen. Außerdem gibt es Hinweise, dass pathologische Potential bei verschiedenen SltI oder SltII-Variaten, bzw. beim Vorkommen beider Gene variiert.

Zur Differenzierung zwischen Sltl und Sltll-Genen können die Primer der Kategorie A bzw. der Kategorien B+C verwendet werden.

Die PCR-Reaktion I) wurde wie folgt angesetzt:
I)
   Probenvolumen - 1 µl
   10xPCR-Puffer - 2,5 µl
   10 mM dNTP - 0,25 µl
   10 µM Vorwärtsprimer
      Kategorie A - 0,2 µl
   10 µM Rückwärtsprimer
      Kategorie A - 0,2 µl
   50 mM MgCl₂ - 0,75 µl
   5U/µl Taq-Polymerase - 0,3 µl
   Wasser - add. 25 µl

   Das obige Reaktionsgemisch wurde in 200µl-Reaktionsgefässen fest verschlossen und gemäss nachfolgendem Protokoll in einem PCR-Gerät inkubiert:
   95° C - 5 min 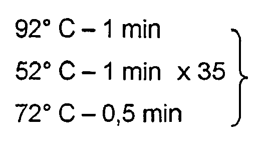
   72° C - 5min

   In dem Reaktionsgemisch wurden jeweils ein Vorwärts- und ein Rückwärtsprimer der Kategorie A (Tab. 1-9) eingesetzt.
   In einer weiteren PCR-Reaktion II) wurde nachfolgendes Gemisch angesetzt:
II)
   Probenvolumen - 1 µl
   10xPCR-Puffer - 2,5 µl
   10 mM dNTP - 0,25 µl
   10 µM Vorwärtsprimer
      Kategorie B+C - 0,2 µl
   10 µM Rückwärtsprimer
      Kategorie B+C - 0,2 µl
   50 mM MgCl₂ - 0,75 µl
   5U/µl Taq-Polymerase - 0,3 µl
   Wasser - add. 25 µl

   Das obige Reaktionsgemisch wurde in 200µl-Reaktlonsgefässen fest verschlossen und gemäss nachfolgendem Protokoll in einem PCR-Gerät inkubiert:
   95° C - 5 min 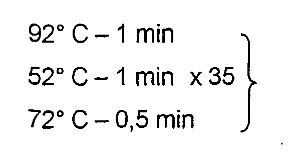
   72° C - 5 min

In dem Reaktionsgemisch wurden jeweils ein Vorwärts- und ein Rückwärtsprimer der Kategorie B+C (Tab. 1-9) eingesetzt.

In der nachfolgenden Tabelle sind die Ergebnisse der PCR-Reaktionen zusammenfasst. Ein positives Ergebnis liegt vor, wenn ein Amplikon, dass eine Bande in der Größenordnung von ca. 500-700 bp amplifiziert wurde. Dieses wurde auf einem Agarosegel, das mit Ethidiumbromid gefärbt wurde sichtbar gemacht.

**Tabelle: Differenzierung zwischen Sltl- und Sltll-Genen**

| | | | | **vorhandene Gene** | | **PCR-Nachweis** | |
|---|---|---|---|---|---|---|---|
| | **BC-Nr** | **Herkunft** | **Serovar** | **SltI** | **SltII** | **Kategorie A** | **Kategorie B+C** |
| 1 | 12502 | Vorzugsmilch | O138H8 | - | + | - | + |
| 2 | 12503 | Vorzugsmilch | O157H- | - | + | - | + |
| 3 | 12504 | Rindfleisch | O8H27 | - | + | - | + |
| 4 | 12505 | Rohmilch | O17H- | - | + | - | + |
| 5 | 12506 | Rinderhackfleisch | O22H- | + | + | + | + |
| 6 | 12507 | Nürnberger Rostbratwurst | O157H- | - | + | - | + |
| 7 | 12508 | Lammfleisch | O84H21 | + | + | + | + |
| 8 | 12509 | Lammfleisch | O7H- | + | + | + | + |
| 9 | 12510 | Lammfleisch | OntH- | + | - | + | - |
| 10 | 12511 | Rinderrohmilchkäse | O23H15 | - | + | - | + |
| 11 | 12512 | Rinderhackfleischrohstoff | O8H- | - | + | - | + |
| 12 | 12513 | Rinderhackfleischrohstoff | O-rauhH23 | + | + | + | + |
| 13 | 12514 | Rinderhackfleischrohstoff | O46H- | - | + | - | + |
| 14 | 12515 | Rinderhackfleisch | O104H12 | + | - | + | - |
| 15 | 12516 | Rinderhackfleisch | O74H- | - | + | - | + |
| 16 | 12517 | Rinderhackfleischrohstoff | O62H8 | + | + | + | + |
| 17 | 12518 | Rinderhackfleischrohstoff | O157H7 | - | + | - | + |
| 18 | 12519 | Pattie aus Rindfleisch | O91 H- | - | + | - | + |
| 19 | 12520 | Rinderhackfleischrohstoff | O22H- | - | + | - | + |
| 20 | 12521 | Zwiebelmettwurst | O65H- | + | - | + | - |
| 21 | 12522 | Rinderhackfleisch | O8H- | - | + | - | + |
| 22 | 12523 | Hackfleisch, gemischt | O91H21 | + | + | + | + |
| 23 | 12524 | Rinderhackfleischrohstoff | O113H4 | - | + | - | + |
| 24 | 12525 | Rinderhackfleisch | O22H8 | + | + | + | + |
| 25 | 12526 | Rinderhackfleischrohstoff | O113H4 | + | + | + | + |

Die Primer der Kategorien A bzw. B+C sind außerdem zu verwenden, um als Konsensusprimer Subtypen der SltI- (Kategorie A) bzw. SltII-Gene (Kategorie B+C) zu amplifizieren. Diese Subtypen können mit spezifischen Sonden, wie sie für die Kategorien A, bzw. B +C aufgelistet sind differenziert werden. Für gegenwärtig noch nicht bekannte Subtypen können die Sonden dieser Kategorien empirisch getestet und den Subtypen zugeordnet werden. Auf Grund der großen Zahl an Sonden ergibt sich ein positiv-negativ-Muster, dass charakteristisch für die Subtypen ist. Außerdem erlauben die Primer der Kategorien A und B+C die Amplifikation und anschließende Sequenzierung der Amplikons. Zudem können Techniken angewendet werden, wie Massenspektrometrie, Hybridisierung auf Biochips, "branch migration inhibition", oder andere Techniken, die eine SNP (single nucleotide polymorphism)-Analyse ermöglichen und dem Fachmann bekannt sind.

### Optimierung einer Online-PCR

Bei einer Online-PCR erfolgt eine simultane Amplifikation und Detektion des Amplikons. Je nachzuweisendem Amplicon werden 1-2 farbstoffmarkierte Sonden dem PCR-Gemisch beigegeben.

Die Detektion der Amplikons kann dann zum Beispiel mit Hilfe eines 5'-Nuklease Assays (TaqMan-Sonden), mit Hilfe von Molecular Beacons, Scorpions Assays oder der zuvor beschriebenen FRET-Technologie erfolgen.

Insbesondere in letzterem Fall ist nur empirisch zu ermitteln, welche der zu verwendenden Sondenpaare optimal geeignet sind. Häufig ist das auftretende Fluoreszenzsignal zu schwach, um ein zuverlässiges und reproduzierbares Ergebnis zu liefern. Außerdem können Sonden in einem komplexen PCR-Gemisch Dimere mit anderen Sonden oder Primern bilden, so dass keine Online-Detektion erfolgt.

Beim Nachweis von EHEC kann es vorteilhaft sein sowohl die Slt-Gene (→ VTEC) als auch die eae-Gene in einer einzigen Multiplex-PCR-Reaktion zu amplifizieren (Slt-Gene + eae-Gen = EHEC) und dann auch simultan zu detektieren. In diesem Fall ist eine sehr präzise Abstimmung der Reaktionskomponenten erforderlich. Durch den Verbrauch der Nukleotide kann die Amplifikation einer von zwei DNA-Zielregionen verhindert werden. Dies bedeutet also, dass die Amplifikation einer DNA durch die Amplifikation einer anderen gequenscht wird. Es ist deshalb notwendig alle Komponenten eines PCR-Gemisches so aufeinander abzustimmen, dass ein Quenschen nicht auftritt.

Dies kann u.a. dadurch geschehen, dass die Primerkonzentrationen limitiert werden. Dabei ist zu berücksichtigen, dass zwischen den Slt-Genen ein Quenschen unproblematisch ist, da der Nachweis nur eines Slt-Genes für die Einstufung als VTEC ausreicht. Aus diesem Grunde können reduzierte Mengen an Sltl- und Sltll-spezifischen Primern beigegeben werden. Die Konzentrationen können im Bereich von 300-200 nM je Primerpaar und PCR-Reaktion liegen. Hingegen sollte die Primerkonzentration der für die eae-Gene höher liegen (310-440 nM), um auch geringe eae-DNA-Konzentrationen in Gegenwart hoher Slt-DNA-Konzentrationen nachweisen zu können.

Eine weitere Möglichkeit ein Quenschen durch die Amplifikation der Slt-Gene zu verhindern besteht darin, eine Annealingtemperatur zu wählen, die Optimal für die eae-spezifischen Primer und suboptimal für die Sltl- und Sltll-spezifischen Primer ist. Konkret kann diese Temperatur bis zu 5° C über der optimalen Temperatur für alle Slt-Primer liegen. Als optimale Temperatur für Primer kann dabei der thermodynamische Schmelzpunkt angesehen werden.

Die Möglichkeiten ein Quenschen zu verhindern sind entsprechend reziprok anwendbar, wenn eae-Gene im Verhältnis zu Sltl- und Sltll-Genen im Überschuss vorliegen oder aus anderen Gründen den Slt-Nachweis quenschen.

Nachfolgend werden PCR-Bedingungen gezeigt, die ein simultanes Amplifizieren der Slt- und eae-Gene erlauben.

Die PCR-Reaktion wird wie folgt angesetzt:
Probenvolumen - 1µl
10 x PCR-Puffer - 2 µl
Stabilisator - 5,53 µl
10 mM dNTP - 0,40 µl
10 - 4 µM Vorwärtsprimer (Stammlsg.)
   SEQ ID Nr. 1, 18, 68 - 0,2 µl
10 - 4 µM Rückwärtsprimer
   SEQ ID Nr. 6, 22, 73 - 0,2 µl
10 µM Sonden SEQ ID Nr. 93, 94, 95, 96, 97, 98, 9, 10, 35, 34
50 mM MgCl₂ - 1,6 µl
1 U/µl Taq-Polymerase - 1 µl
Wasser - add. 20 µl

### Temperaturzyklen im Lightcycler:

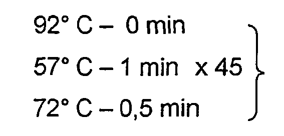

72° C - 5 min

Figur 4 zeigt die Amplifikation von Sltl- und Sltll-Genen durch Realtime-PCR. Verwendet wurden Sonden, die die Detektion sowohl der SltI- als auch der SltII-Gene erlauben. Diese wurden jeweils mit gleichen Fluoreszenzfarbstoffen (Lighcycler RED 640 und Fluorescein) gekoppelt, so dass die Detektion in nur einem Kanal (F2) erfolgt. Es ist zu erkennen, dass bei der Amplifikation der SltII-Gene Signalkurven mit Amplituden entstehen, die größer als 14 sind. Die Signalkurven der SltI-Gene liegen signifikant tiefer. Wenn sowohl SltI- als auch SltII-Gene vorkommen, dann weist die Amplitude das höchste Niveau auf. Sie ist somit als Indikator für das Vorkommen und die Differenzierung zwischen den SltI- und SltII-Genen geeignet.

Aus Figur 4 ist auch zu sehen, dass je nach Verwendung verschiedener Sonden die Signalamplitude für die SltI-Gene unterschiedlich hoch ist. In der Abbildung wurden die Sonden Nr.9+10 (Stamm Nr. 1-10), Nr. 95+96 (Stamm Nr. 11-20), Nr. 97+98 (Stamm Nr. 21-30) und Sonden Nr. 34+35 (Stamm Nr. 1-30) zusammen mit den Primern Nr. 1+6 und 18+22 verwendet. Außerdem sind in der PCR-Mixtur die Oligonukleotide für den Nachweis der eae-Gene (s. nachfolgend) vorhanden.

Das eae-Gen wurde mit Sonden, die mit den Fluoreszenzfarbstoffen Lightcycler RED 705 und Fluorescein gekoppelt sind, nachgewiesen. Ihre Detektion erfolgte somit in einem anderen Kanal (F3) als die der Slt-Gene (F2). Für den eae-Nachweis wurden die Sonden Nr. 93+94 und die Primer Nr. 68+73 verwendet. Es ist in Figur 5 zu erkennen, dass alle eae-positiven Stämme Signalamplituden erzeugen, die größer als 5 sind.

**Tabelle: Vorkommen der Pathogenitätsgene bei den in der Realtime-PCR verwendeten VTEC-/EHEC-Stämmen**

| **Stamm-Nr. in Fig. 4, 5** | **SltI** | **SltII** | **eae** |
|---|---|---|---|
| 2, 12, 22 | - | + | + |
| 3, 13, 23 | - | + | + |
| 4, 14, 24 | + | + | - |
| 5, 15, 25 | - | + | - |
| 6,16,26 | + | - | + |
| 7,17,27 | + | - | + |
| 8,18,28 | + | - | + |
| 9,19,29 | + | - | + |
| 10,20,30 | + | - | + |

Stämme in der gleichen Zeile in obiger Tabelle
sind jeweils identisch (d.h. 2=12=22)

Als Gegenstand dieser Erfindung werden Oligonukleotide bereitgestellt, die besonders gut für den Nachweis von EHEC oder VTEC geeignet sind. Innerhalb der Menge dieser Oligonukleotide gibt es einige, die sich besonders gut für diesen Nachweis eignen. Diese sind in der nachfolgenden Tabelle zusammengefaßt.

Tabelle: bevorzugte Oligonukleotidkombinationen für den Nachweis von pathogenen E.coli

| nachzuweisende Organismen | Primer | Sonden |
|---|---|---|
| VTEC | Nr. 1+6+18+22 | 9+10, 95+96, 97+98, 34+35 |
| VTEC | Nr. 1+6+18+22+84+85+86+87 | 9+10, 95+96, 97+98, 34+35, 89+90 |
| EHEC (s. Fig. 4+5) | Nr. 1+6+18+22, 68+73 | 9+10, 95+96, 97+98, 34+35, 93+94 |
| EHEC | Nr. 1+6+18+22, 68+73+84+85+86+87 | 9+10, 95+96, 97+98, 34+35, 93+94,89+90 |
| EHEC | Nr. 1+6+18+22+46+54 | 9+10, 95+96, 97+98, 34+35, 60+61 |
| EHEC | Nr. 1+6+18+22, 68+73+84+85+86+87+46+54 | 9+10, 95+96, 97+98, 34+35, 93+94,89+90+60+61 |

Sofern eine Detektion nur durch Visualisierung der Amplikons im Agarosegel erfolgt, können in der Multiplex-Mixtur die Sonden aus obiger Tabelle fortgelassen werden.

**Tabelle: Optimierung der Realtime EHEC-PCR**

| **Problem** | **Lösung** |
|---|---|
| Spezifizierung als EHEC | Simultane Amplifikation der Sltl/ll-Gene und eines eae-Gens oder Nachweis in zwei PCR-Schritten; ggfalls. |
| | Nachweis der Spezies Escherichia coli zusätzlich zu den Pathogenitätsgenen |
| Spezifizierung als EHEC | Simultane Amplifikation der SItI/II-Gene und des hlyA-Gens oder Nachweis in zwei PCR-Schritten; ggfalls; |
| | Nachweis der Spezies Escherichia coli zusätzlich zu den Pathogenitätsgenen |
| Spezifizierung als EHEC | Simultane Amplifikation der Sltl/II-Gene und des eae-Gens und des hlyA-Gens oder Nachweis in drei PCR-Schritten; ggfalls. |
| | Nachweis der Spezies Escherichia coli zusätzlich zu den Pathogenitätsgenen |
| verschiedene Slt-Gene werden mit gleichen Fluoreszensfarbstoffen nachgewiesen | Sltl- und Sltll-Gene lassen sich durch den Kurvenverlauf und die Höhe der Amplitude differenzieren. Weitere Differenzierung durch Schmelzkurvenanalyse möglich |
| Die simultane Amplifikation der Slt- und eae- und/oder hlyA-Gene wird gequenscht | Primer werden limitiert |
| Die Amplifikation der Slt- und eae- und/oder hlyA-Gene wird gequenscht | Annealingtemperaturen der Primer und/oder Sonden werden bezüglich Quenschen optimal gewählt. |
| Die Amplifikation der Slt- und eae- und/oder hlyA-Gene wird gequenscht | Auswahl der Sonden und Primer reduziert quenschen signifikant. Die Amplifikationseffizienz wird durch diese Oligonukleotide maßgeblich beeinflußt . Deshalb wurden die Primer und Sonden harmonisch aufeinander abgestimmt. |
| Die Signalhöhe bei Sonden ist zu gering | Testen einer großen Anzahl von Sonden/Sondenpaaren und empirische Auswahl der besten Sonden |

## Patentansprüche

1. Verfahren zum Nachweis von EHEC-Bakterien in einer Probe, umfassend den Schritt:
Nachweisen des Vorkommens einer Nukleinsäuresequenz aus dem Slt-Locus in Kombination mit einer Sequenz aus dem eae-Locus und/oder dem hylA-Locus in der Probe,
wobei zumindest zwei Vorwärtsprimer/Rückwärtsprimer-Paare, welche SEQ ID Nrn: 1 und 6 (Kategorie A) oder jeweils entsprechende Derivate davon, bzw. SEQ ID Nm: 18 und 22 (Kategorie B) oder jeweils entsprechende Derivate davon sind, kombiniert werden mit zumindest einem Vorwärtsprimer und zumindest einem Rückwärtsprimer aus einer der Kategorien D, d.h. ausgewählt aus SEQ ID Nr: 46 oder entsprechenden Derivaten davon (Vorwärtsprimer) bzw. SEQ ID Nr: 54 oder entsprechenden Derivaten davon (Rückwärtsprimer), und E, d.h. ausgewählt aus SEQ ID Nrn: 68, oder entsprechenden Derivaten davon (Vorwärtsprimer) bzw. SEQ ID Nm: 73 oder entsprechenden Derivate davon (Rückwärtsprimer),
wobei die entsprechenden Derivate
a) zumindest 80% identisch zur jeweiligen oben aufgeführten Sequenz sind, oder
b) sich durch genau einen Basenaustausch, genau eine Insertion, genau eine Deletion oder genau eine Addition von einer jeweiligen Sequenz unterscheiden; und
wobei der gleichzeitige Nachweis von Sequenzen
i) aus dem Slt-Locus und dem eae-Locus oder
ii) aus dem Slt-Locus und dem hlyA-Locus
in der Probe eine ausreichend hohe Aussagekraft für den EHEC Nachweis besitzt.

2. Verfahren nach Anspruch 1, **dadurch gekennzeichnet, dass** der Nachweis mindestens eine PCR umfasst.

3. Verfahren gemäß Anspruch 1, wobei die Primerpaare aus einer der Kategorie D oder E ausgewählt sind aus den Paaren von
a) SEQ ID NOS: 68 und 73 (Kategorie E) oder entsprechenden Derivaten davon oder
b) SEQ ID NOS: 46 und 54 (Kategorie D) oder entsprechenden Derivaten davon,
wobei die entsprechenden Derivate
(i) zumindest 80% identisch zur jeweiligen oben aufgeführten Sequenz sind, oder
(ii) sich durch genau einen Basenaustausch, genau eine Insertion, genau eine Deletion oder genau eine Addition von einer jeweiligen Sequenz unterscheiden.

4. Verfahren nach einem der Ansprüche 1 bis 3, **dadurch gekennzeichnet, dass** ein zusätzliches Oligonukleotid umfassend mindestens eine Sequenz ausgewählt aus einer der SEQ ID Nm: 84-92 (Kategorie F) und Derivaten hiervon verwendet wird,
wobei die entsprechenden Derivate
(i) zumindest 80% identisch zur jeweiligen oben aufgeführten Sequenz sind, oder
(ii) sich durch genau einen Basenaustausch, genau eine Insertion, genau eine Deletion oder genau eine Addition von einer jeweiligen Sequenz unterscheiden.

5. Verfahren nach einem der Ansprüche 1 bis 4, **dadurch gekennzeichnet, dass** mehrere Oligonukleotide im Rahmen einer Multiplex-PCR oder in mindestens 2 getrennten, sequentiellen PCR eingesetzt werden.

6. Verfahren nach einem der Ansprüche 1 bis 5, **dadurch gekennzeichnet, dass** der Nachweis das Inkontaktbringen der Nukleinsäure aus der Probe, gegebenenfalls nach deren Amplifikation, mit einem Biochip, enthaltend die Oligonukleotide zum Nachweis von EHEC, umfasst.

7. Verfahren nach einem der Ansprüche 1 bis 6, **dadurch gekennzeichnet, dass** es mindestens einen weiteren Schritt umfasst, ausgewählt aus
- Amplifikation der nachzuweisenden Nukleinsäure;
- PCR-Amplifikation der nachzuweisenden Nukleinsäure;
- Southemblothybridisierung der nachzuweisenden Nukleinsäure mit geeigneten Sonden, vorzugsweise ausgewählt aus einer Nukleinsäure umfassend mindestens eine Sequenz mit einer der SEQ ID NOS: 1 bis 98;
- Ligase-Kettenreaktion mit der nachzuweisenden Nukleinsäure; und
- isotherme Nukleinsäureamplifikation der nachzuweisenden Nukleinsäure.

8. Verfahren nach einem der Ansprüche 1 bis 7, **dadurch gekennzeichnet, dass** der Nachweis eine Online-Detektion erhaltener Amplikons umfasst.

9. Verfahren nach einem der Ansprüche 1 bis 8, **dadurch gekennzeichnet, dass** die Amplifikation und/oder Detektion der nachzuweisenden Nukleinsäure auf einem Biochip erfolgt.

10. Eine Kombination von Oligonukleotiden umfassend
zumindest zwei Primerpaare von
Vorwärts- und Rückwärtsprimern, ausgewählt aus
A) SEQ ID Nrn:1 und 6 (Kategorie A) oder jeweils entsprechenden Derivaten davon, und
B) SEQ ID NOS: 18 und 22 (Kategorie B) oder jeweils entsprechenden Derivaten davon, und
zumindest ein Primerpaar von Vorwärts- und Rückwärtsprimern, ausgewählt aus einer der Kategorien
i) D, d.h. ausgewählt aus SEQ ID Nm: 46 oder entsprechenden Derivaten davon (Vorwärtsprimer), bzw. SEQ ID Nrn: 54 oder entsprechenden Derivaten davon (Rückwärtsprimer), und
ii) E, d.h. ausgewählt aus SEQ ID Nr: 68 oder entsprechenden Derivaten davon (Vorwärtsprimer), bzw. SEQ ID Nr: 73 oder entsprechenden Derivaten davon (Rückwärtsprimer),
vorzugsweise ein Primerpaar von D und ein Primerpaar von E,
wobei die entsprechenden Derivate
a) zumindest 80% identisch zur jeweiligen oben aufgeführten Sequenz sind, oder
b) sich durch einen Basenaustausch, eine Insertion, Deletion oder Addition von einer jeweiligen Sequenz unterscheiden.

11. Kombination nach Anspruch 10, **dadurch gekennzeichnet, dass** sie weiterhin ein Oligonukleotid umfassend mindestens eine Sequenz ausgewählt aus der Kategorie F umfasst.

12. Kit zum Nachweis von EHEC-Bakterien enthaltend eine Kombination nach einem der Ansprüche 10 oder 11.

13. Verwendung einer Kombination nach Anspruch 10 oder 11 zum Nachweis von EHEC-Bakterien.

## Claims

1. A method for detecting EHEC bacteria in a sample, comprising the step:
detecting the occurrence of a nucleic acid sequence from the Slt locus in combination with a sequence from the eae locus and/or the hlyA locus in the sample,
wherein at least two forward primer/backward primer pairs, which are SEQ ID nos. 1 and 6 (category A) or in each case corresponding derivatives thereof, or SEQ ID nos. 18 and 22 (category B) or in each case corresponding derivatives thereof, are combined with at least one forward primer and at least one backward primer from one of categories D, i.e. selected from SEQ ID no. 46 or corresponding derivatives thereof (forward primer) or SEQ ID no. 54 or corresponding derivatives thereof (backward primer), and E, i.e. selected from SEQ ID no. 68, or corresponding derivatives thereof (forward primer) or SEQ ID no. 73 or corresponding derivatives thereof (backward primer),
wherein the corresponding derivatives
a) are at least 80% identical to the respective sequence listed above, or
b) differ from a respective sequence by exactly one base substitution, exactly one insertion, exactly one deletion or exactly one addition; and
wherein simultaneous detection of sequences
i) from the Slt locus and the eae locus or
ii) from the Slt locus and the hlyA locus
in the sample has a sufficiently high level of predictiveness for EHEC detection.

2. A method according to claim 1, **characterised in that** detection comprises at least one PCR.

3. A method according to claim 1, wherein the primer pairs from one of categories D or E are selected from the pairs
a) SEQ ID nos. 68 and 73 (category E) or corresponding derivatives thereof or
b) SEQ ID nos. 46 and 54 (category D) or corresponding derivatives thereof,
wherein the corresponding derivatives
i) are at least 80% identical to the respective sequence listed above, or
ii) differ from a respective sequence by exactly one base substitution, exactly one insertion, exactly one deletion or exactly one addition.

4. A method according to any one of claims 1 to 3, **characterised in that** an additional oligonucleotide comprising at least one sequence selected from one of SEQ ID nos. 84-92 (category F) and derivatives thereof is used,
wherein the corresponding derivatives
i) are at least 80% identical to the respective sequence listed above, or
ii) differ from a respective sequence by exactly one base substitution, exactly one insertion, exactly one deletion or exactly one addition.

5. A method according to any one of claims 1 to 4, **characterised in that** two or more oligonucleotides are used in the context of a multiplex PCR or in at least 2 separate, sequential PCRs.

6. A method according to any one of claims 1 to 5, **characterised in that** detection involves bringing the nucleic acid from the sample, optionally after amplification thereof, into contact with a biochip containing the oligonucleotides for detecting EHEC.

7. A method according to any one of claims 1 to 6, **characterised in that** it comprises at least one further step selected from
- amplification of the nucleic acid to be detected;
- PCR amplification of the nucleic acid to be detected;
- Southern blot hybridisation of the nucleic acid to be detected with suitable probes, preferably selected from a nucleic acid comprising at least one sequence with one of SEQ ID nos. 1 to 98;
- ligase chain reaction with the nucleic acid to be detected; and
- isothermal nucleic acid amplification of the nucleic acid to be detected.

8. A method according to any one of claims 1 to 7, **characterised in that** detection comprises online detection of amplicons which have been obtained.

9. A method according to any one of claims 1 to 8, **characterised in that** amplification and/or detection of the nucleic acid to be detected proceeds on a biochip.

10. A combination of oligonucleotides comprising
at least two primer pairs of
forward and backward primers, selected from
A) SEQ ID nos. 1 and 6 (category A) or in each case corresponding derivatives thereof, and
B) SEQ ID nos. 18 and 22 (category B) or in each case corresponding derivatives thereof, and
at least one primer pair of forward and backward primers, selected from one of categories
i) D, i.e. selected from SEQ ID no. 46 or corresponding derivatives thereof (forward primer), or SEQ ID no. 54 or corresponding derivatives thereof (backward primer), and
ii) E, i.e. selected from SEQ ID no. 68 or corresponding derivatives thereof (forward primer), or SEQ ID no. 73 or corresponding derivatives thereof (backward primer),
preferably one primer pair of D and one primer pair of E,
wherein the corresponding derivatives
a) are at least 80% identical to the respective sequence listed above, or
b) differ from a respective sequence by a base substitution, insertion, deletion or addition.

11. A combination according to claim 10, **characterised in that** it furthermore comprises an oligonucleotide comprising at least one sequence selected from category F.

12. A kit for detecting EHEC bacteria containing a combination according to either claim 10 or claim 11.

13. Use of a combination according to claim 10 or claim 11 for detecting EHEC bacteria.

## Revendications

1. Procédé de détection de bactéries EHEC dans un échantillon, comprenant l'étape consistant à :
détecter l'apparition d'une séquence d'acide nucléique provenant du locus Slt en combinaison avec une séquence provenant du locus eae et/ou du locus hylA dans l'échantillon,
où au moins deux paires amorce sens/amorce antisens, qui sont les SEQ ID N°: 1 et 6 (catégorie A) ou les dérivés respectivement correspondants de celles-ci ou les SEQ ID N°: 18 et 22 (catégorie B) ou les dérivés respectivement correspondants de celles-ci, sont combinées avec au moins une amorce sens et au moins une amorce antisens issue d'une des catégories D, c'est-à-dire choisie parmi la SEQ ID N°: 46 ou les dérivés correspondants de celle-ci (amorce sens) ou la SEQ ID N°: 54 ou les dérivés correspondants de celle-ci (amorce antisens), et E, c'est-à-dire choisie parmi la SEQ ID N°: 68 ou les dérivés correspondants de celle-ci (amorce sens) ou la SEQ ID N°: 73 ou les dérivés correspondants de celle-ci (amorce antisens),
où les dérivés correspondants
a) sont au moins à 80 % identiques avec la séquence respective exposée ci-dessus, ou
b) se distinguent d'une séquence respective par exactement un échange de bases, exactement une insertion, exactement une délétion ou exactement une addition ; et
où la détection simultanée des séquences
i) provenant du locus Slt et du locus eae
ii) provenant du locus Slt et du locus hylA
dans l'échantillon possède une pertinence suffisamment élevée pour la détection des EHEC.

2. Procédé selon la revendication 1, **caractérisé en ce que** la détection comprend au moins une PCR.

3. Procédé selon la revendication 1, où les paires d'amorces provenant d'une des catégories D ou E sont choisies parmi les paires de
a) SEQ ID N°: 68 et 73 (catégorie E) ou les dérivés correspondants de celles-ci ou
b) SEQ ID N°: 46 et 54 (catégorie D) ou les dérivés correspondants de celles-ci
où les dérivés correspondants
i) sont au moins à 80 % identiques avec la séquence respective exposée ci-dessus, ou
ii) se distinguent d'une séquence respective par exactement un échange de bases, exactement une insertion, exactement une délétion ou exactement une addition.

4. Procédé selon l'une des revendications 1 à 3, **caractérisé en ce que** l'on utilise un oligonucléotide supplémentaire comprenant au moins une séquence choisie parmi une des SEQ ID N°: 84 - 92 (catégorie F) et leurs dérivés,
où les dérivés correspondants
i) sont au moins à 80 % identiques avec la séquence respective exposée ci-dessus, ou
ii) se distinguent d'une séquence respective par exactement un échange de bases, exactement une insertion, exactement une délétion ou exactement une addition.

5. Procédé selon l'une des revendications 1 à 4, **caractérisé en ce que** plusieurs oligonucléotides sont mis en oeuvre dans le cadre d'une PCR multiplex ou dans au moins 2 PCR séquentielles séparées.

6. Procédé selon l'une des revendications 1 à 5, **caractérisé en ce que** la détection comprend la mise en contact de l'acide nucléique provenant de l'échantillon, le cas échéant après son amplification, avec une biopuce, contenant les oligonucléotides pour la détection des EHEC.

7. Procédé selon l'une des revendications 1 à 6, **caractérisé en ce qu'**il comprend au moins une autre étape, choisie parmi :
- l'amplification de l'acide nucléique à détecter ;
- l'amplification par PCR de l'acide nucléique à détecter ;
- l'hybridation par Southern blot de l'acide nucléique à détecter avec des sondes appropriées, de préférence choisies à partir d'un acide nucléique comprenant au moins une séquence ayant l'une des SEQ ID N°: 1 à 98 ;
- la réaction en chaîne ligase avec l'acide nucléique à détecter; et
- l'amplification isotherme de l'acide nucléique à détecter.

8. Procédé selon l'une des revendications 1 à 7, **caractérisé en ce que** la détection comprend une détection en ligne des amplicons obtenus.

9. Procédé selon l'une des revendications 1 à 8, **caractérisé en ce que** l'amplification et/ou la détection de l'acide nucléique à détecter se fait sur une biopuce.

10. Combinaison d'oligonucléotides, comprenant :
au moins deux paires d'amorces d'
amorces sens et antisens, choisies parmi :
A) les SEQ ID N°: 1 et 6 (catégorie A) ou les dérivés respectivement correspondants de celles-ci et
B) les SEQ ID N°: 18 et 22 (catégorie B) ou les dérivés respectivement correspondants de celles-ci, et
au moins une paire d'amorces d'amorce sens et amorce antisens, choisies dans l'une des catégories
i) D, c'est-à-dire choisie parmi la SEQ ID N°: 46 ou les dérivés correspondants de celle-ci (amorce sens) ou la SEQ ID N°: 54 ou les dérivés correspondants de celle-ci (amorce antisens), et
ii) E, c'est-à-dire choisie parmi la SEQ ID N°: 68 ou les dérivés correspondants de celle-ci (amorce sens) ou la SEQ ID N°: 73 ou les dérivés correspondants de celle-ci (amorce antisens),
de préférence une paire d'amorces de D et une paire d'amorces de E,
où les dérivés correspondants
a) sont au moins à 80 % identiques avec la séquence respective exposée ci-dessus, ou
b) se distinguent d'une séquence respective par exactement un échange de bases, exactement une insertion, exactement une délétion ou exactement une addition.

11. Combinaison selon la revendication 10, **caractérisée en ce qu'**elle comprend en outre un oligonucléotide contenant au moins une séquence choisie dans la catégorie F.

12. Kit de détection des bactéries EHEC contenant une combinaison selon l'une des revendications 10 ou 11.

13. Utilisation d'une combinaison selon la revendication 10 ou 11 pour la détection des bactéries EHEC.
